# FASCICULE DE BREVET EUROPEEN

(11) **EP 1 109 818 B1**
(45) Date de publication et mention de la délivrance du brevet: **06.11.2002**
(21) Numéro de dépôt: 99940246.4
(22) Date de dépôt: 27.08.1999
(51) Int. Cl.: C07F 9/655, C07H 19/10, C12N 5/06, A61K 31/665, A61K 49/00

(54) **PHOSPHOEPOXYDES, PROCEDE DE FABRICATION ET APPLICATIONS**
PHOSPHOEPOXIDE, VERFAHREN ZU DEREN HERSTELLUNG UND IHRE VERWENDUNGEN
PHOSPHOEPOXIDES, METHOD FOR MAKING SAME AND USES

(30) Priorité: 01.09.1998 FR 9810914
(43) Date de publication de la demande: 27.06.2001
(73) Titulaire: INSTITUT NATIONAL DE LA SANTE ET DE LA RECHERCHE MEDICALE (INSERM), 75013 Paris (FR)
(72) Inventeur: BELMANT, Christian, F-31700 Blagnac (FR); FOURNIE, Jean-Jacques, F-31450 Corronsac (FR); BONNEVILLE, Marc, F-44120 Vertou (FR); PEYRAT, Marie-Alix, F-44230 Saint-Sebastien sur Loire (FR)
(74) Mandataire: Cabinet BARRE LAFORGUE & associés
(86) Numéro de dépôt international: FR9902057
(87) Numéro de publication internationale: WO00012519

(56) Documents cités:
- WO-A-95/20673
- US-A- 5 639 653
- MUEHLBACHER M: "Isopentenyl-diphosphate isomerase: inactivation of the enzyme with active-site-directed irreversible inhibitors and transition-state analogues" BIOCHEMISTRY., vol. 27, no. 19, 1988, pages 7315-7328, XP002102417 EASTON, PA US cité dans la demande
- TANAKA Y ET AL: "Natural and synthetic non-peptide antigens recognized by human.gamma..delta. T cells" NATURE (LONDON) (NATUAS,00280836);1995; VOL.375 (6527); PP.155-8, XP002102418 Albert Einstein College Medicine;Howard Hughes Medical Inst.; Bronx; 10461; NY; USA (US)
- WESCH D ET AL: "Comparative analysis of.alpha..beta. and.gamma..delta. T cell activation by Mycobacterium tuberculosis and isopentenyl pyrophosphate" EUR. J. IMMUNOL. (EJIMAF,00142980);1997; VOL.27 (4); PP.952-956, XP002102419 Paul Ehrlich Institute;Department Immunology; Langen; D-63225; Germany (DE)
- BUERK M R ET AL: "Human V.gamma.9-V.delta.2 cells are stimulated in a cross-reactive fashion by a variety of phosphorylated metabolites" EUR. J. IMMUNOL. (EJIMAF,00142980);1995; VOL.25 (7); PP.2052-8, XP002102420 University Hospital;Experimental Immunology, Department of Research; Basel; CH-4031; Switz. (CH)
- SCHOEL B ET AL: "Phosphate is essential for stimulation of V.gamma.9V.delta.2 T lymphocytes by mycobacterial low molecular weight ligand" EUR. J. IMMUNOL. (EJIMAF,00142980);1994; VOL.24 (8); PP.1886-92, XP002102421 University of Ulm;Department of Immunology; Ulm; Germany (DE)

## Description

L'invention concerne de nouveaux composés phosphoépoxydes, leur procédé de fabrication et leurs applications pour la stimulation des lymphocytes Tγ9δ2 porteurs de récepteurs TCR à régions variables Vγ9 et Vδ2.

Les lymphocytes Tγδ des primates présents dans le sang périphérique (humains, singes) représentent, chez l'individu sain, habituellement de 1 à 5% des lymphocytes du sang et jouent un rôle dans le système immunitaire. Il a été démontré qu'ils reconnaissent leurs ligands antigéniques par une interaction directe avec l'antigène, sans présentation par les molécules du CMH d'une cellule présentatrice. Les lymphocytes Tγ9δ2 (parfois aussi désignés lymphocytes Tγ262) sont des lymphocytes Tγδ porteurs de récepteurs TCR à régions variables Vγ9 et Vδ2. Ils représentent la majorité des lymphocytes Tγδ dans le sang humain.

Lorsqu'ils sont activés, les lymphocytes Tγδ exercent une puissante activité cytotoxique non restreinte par le CMH, particulièrement efficace pour tuer divers types de cellules, notamment des cellules pathogènes. Il peut s'agir de cellules infectées par des virus ("γδ T cell activation or anergy during infections : the rôle of nonpeptidic TCR ligands and HLA class I molécules" Fabrizio POCCIA *et al.* Journal of Leukocyte Biology, 62, 1997, p. 1-5), ou par d'autres parasites intracellulaires tels que les mycobactéries ("The antituberculous *Mycobacterium bovis* BCG Vaccine is an attenuated Mycobacterial producer of phosphorylated nonpeptidic Antigens for human γδ T cells" Patricia CONSTANT *et al.* Infection and Immunity, vol. 63, n° 12, Dec. 1995, p. 4628-4633) ; ou les protozoaires *("Plasmodium falciparum* stimuli for human γδ T Cells are related to phosphorylated Antigens of mycobacteria" Charlotte BEHR *et al.* Infection and Immunity, Vol. 64, n° 8, 1996, p. 2892-2896). Il peut aussi s'agir de cellules cancéreuses ("CD94/NKG2 inhibitory receptor complex modulates both antiviral and antitumoral responses of polyclonal phosphoantigen-reactive Vγ9 Vδ2 T lymphocytes" Fabrizio POCCIA *et al,* Journal of Immunology, 159, p. 6009-6015 ; "Stimulation of γδ T cells by phosphoantigens" Jean-Jacques FOURNIE, Marc BONNEVILLE, Res. Immunol., 66^{th} FORUM IN IMMUNOLOGY, 147, P. 338-347).

Il a été démontré que les lymphocytes Tγ9δ2 humains réagissent dans le cas d'une infection mycobactérienne à quatre molécules naturelles non peptidiques de structure phosphatée, désignées phosphoantigènes, qui présentent une activité de stimulation pour une concentration de l'ordre de 1 à 5 nM (nanomolaire) (WO-95/20673 et "Stimulation of human γδ T cells by nonpeptidic Mycobacterial ligands" Patricia CONSTANT *et al.* Science, 264, p. 267-270).

Ces antigènes naturels ne sont pas complètement identifiés. Certains auteurs les ont présentés, à tort, comme des dérivés alcènes du pyrophosphate, notamment l'isopentènyl pyrophosphate IPP (US-5 639 653 et "Natural and Synthetic nonpeptide antigens recognized by human γδ T cells", Yoshimasa TANAKA *et al.* Nature, 375, 1995, p. 155-158). Néanmoins, il est maintenant démontré qu'aucun de ces prénylpyrophosphates n'est actif à une concentration de l'ordre du nanomolaire. Les meilleurs résultats obtenus n'arrivent pas à démontrer une activité à moins de 3 µM pour l'IPP, et à 0,3 µM pour le diméthylallyl-UTP et le 3-méthyl-2-hexène pyrophosphate. La concentration minimale d'activité de ces composés est donc, au mieux, de l'ordre de 100 fois plus importante que celle des phosphoantigènes naturels.

En ce qui concerne l'IPP, il est à noter en particulier que les dernières publications mentionnées ci-dessus commettent une erreur en déduisant la structure du radical isopentènyl à partir de la seule analyse du spectre de masse et de la mise en évidence d'une certaine bioactivité. En effet, outre le fait que le composé analysé dans les publications n'était pas purifié et qu'un spectre de masse ne peut pas identifier des espèces non chargées, on peut démontrer qu'il existe en fait plusieurs milliers de structures chimiques différentes pouvant avoir cette même masse moléculaire et être un substituant du pyrophosphate dans ces molécules.

Le fait que la concentration minimale d'activité de l'IPP soit beaucoup plus élevée (de l'ordre de 1000 fois) et que l'intensité des réponses lymphocytaires Tγ9δ2 obtenues soit beaucoup plus faible que celles des phosphoantigènes naturels démontre que l'IPP n'est pas l'un de ces phosphoantigènes naturels ("A novel nucleotide-containing antigen for human blood γδ T lymphocytes", Y. Poquet *et al,* Eur. J. Immunol. 1996, 26, p. 2344-2349). Cela est d'ailleurs confirmé par de nombreuses autres constatations : on ne trouve pas d'IPP en concentration suffisante dans les extraits mycobactériens stimulant les lymphocytes Tγ9δ2 ; l'IPP n'a pas les mêmes caractéristiques chromatographiques (HPAEC) selon : "High pH anion exchange chromatographic analysis of phosphorylated compounds : application to isolation and characterization of non peptide mycobacterial antigens", Y. Poquet *et al,* Anal. Biochem, 243 n° 1, 1996, p. 119-126, que les phosphoantigènes naturels ; l'IPP et les autres isoprénoïdes naturels sont produits par toutes les cellules vivantes, qui ne stimulent pourtant pas les lymphocytes Tγ9δ2.

Par ailleurs, on sait que les substances dont la bioactivité est de l'ordre de ou supérieure à 1 µM ne sont que rarement compatibles avec les contraintes de rentabilité d'une exploitation à l'échelle industrielle. Ainsi, les phosphoantigènes synthétiques proposés jusqu'à maintenant ne sont pas exploitables à l'échelle industrielle dans des conditions économiques acceptables.

Les phosphoantigènes naturels, quant à eux, ne peuvent être produits qu'en très faibles quantités (WO 95/20673), et leur structure chimique exacte restant encore indéterminée, il n'est pas possible de les produire par synthèse. Il n'est donc pas non plus possible d'envisager une exploitation à l'échelle industrielle dans des conditions économiques, malgré leur grand intérêt thérapeutique démontré.

L'invention vise donc à proposer des nouveaux composés chimiques qui soient activateurs des lymphocytes Tγ9δ2 pour une concentration minimale d'activation inférieure à 100nM, notamment de l'ordre de 10nM.

L'invention vise aussi à proposer des composés pouvant être couplés à un grand nombre de groupements organiques, notamment à des groupements peptidiques naturels ou synthétiques, de façon à permettre l'obtention de composés multifonctionnels.

L'invention vise aussi à proposer un procédé de fabrication de composés selon l'invention.

L'invention vise aussi à proposer des applications selon l'invention à titre d'activateurs des lymphocytes Tγ9δ2, et en particulier des applications thérapeutiques des composés selon l'invention.

L'invention vise aussi à proposer de tels composés dont la synthèse est simple, quantitative et peu coûteuse, c'est-à-dire compatible avec les contraintes économiques d'une production à l'échelle industrielle.

L'invention vise aussi à ce titre à proposer une voie de synthèse avantageuse de ces composés.

L'invention vise aussi à proposer un procédé de fabrication de composés selon l'invention.

L'invention vise aussi à proposer des applications des composés selon l'invention à titre d'activateur des lymphocytes Tγ9δ2, et en particulier des applications thérapeutiques des composés selon l'invention.

L'invention concerne donc des composés comprenant au moins un groupement phosphoépoxyde de formule : 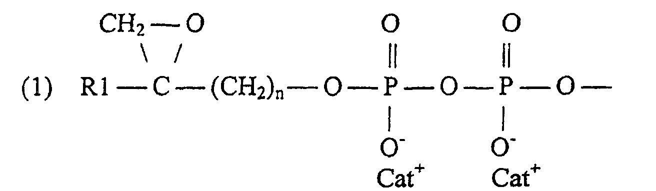 où R1 est choisi parmi ―CH₃ et ―CH₂ ―CH₃,
Cat⁺ représente un (ou des) cation(s) organique(s) ou minéral(aux) (y compris le proton) identiques ou différents dans le même composé,
et n est un nombre entier compris entre 2 et 20.

Un composé selon l'invention peut comprendre notamment un ou plusieurs groupement(s) époxyde(s) choisi(s) parmi les esters des groupements suivants (nomenclature IUPAC), ou parmi les composés formés de ces groupements :
3,4-époxy-3-méthyl-1-butyl-diphosphate ; 3,4-époxy-3-éthyl-1-butyl-diphosphate ; 4,5-époxy-4-méthyl-1-pentyl-diphosphate ; 4,5-époxy-4-éthyl-1-pentyl-diphosphate ; 5,6-époxy-5-méthyl-1-hexyl-diphosphate ; 5,6-époxy-5-éthyl-1-hexyl-diphosphate ; 6,7-époxy-6-méthyl-1-heptyl-diphosphate ; 6,7-époxy-6-éthyl-1-heptyl-diphosphate ; 7,8-époxy-7-méthyl-1-octyl-diphosphate; 7,8-époxy-7-éthyl-1-octyl-diphosphate; 8,9-époxy-8-méthyl-1-nonyl-diphosphate ; 8,9-époxy-8-éthyl-1-nonyl-diphosphate ; 9,10-époxy-9-méthyl-1-décyl-diphosphate ; 9,10-époxy-9-éthyl-1-décyl-diphosphate ; 10,11-époxy-10-méthyl-1-undécyl-diphosphate ; 10,11-époxy-10-éthyl-1-undécyl-diphosphate ; 11,12-époxy-11-méthyl-1-dodécyl-diphosphate ; 11,12-époxy-11-éthyl-1-dodécyl-diphosphate; 12,13-époxy-12-méthyl-1-tridécyl-diphosphate; 12,13-époxy-12-éthyl-1-tridécyl-diphosphate; 13,14-époxy-13-méthyl-1-tétradécyl-diphosphate; 13,14-époxy-13-éthyl-1-tétradécyl-diphosphate ; 14,15-époxy-14-méthyl-1-pentadécyl-diphosphate; 14,15-époxy-14-éthyl-1-pentadécyl-diphosphate; 15,16-époxy-15-méthyl-1-hexadécyl-diphosphate ; 15,16-époxy-15-éthyl-1-hexadécyl-diphosphate ; 16,17-époxy-16-méthyl-1-heptadécyl-diphosphate; 16,17-époxy-16-éthyl-1-heptadécyl-diphosphate ; 17,18-époxy-17-méthyl-1-octadécyl-diphosphate; 17,18-époxy-17-éthyl-1-octadécyl-diphosphate ; 18,19-époxy-18-méthyl-1-nonadécyl-diphosphate ; 18,19-époxy-18-éthyl-1-nonadécyl-diphosphate; 19,20-époxy-19-méthyl-1-eicosyl-diphosphate ; 19,20-époxy-19-éthyl-1-eicosyl-diphosphate; 20,21-époxy-20-méthyl-1-heneicosyl-diphosphate ; 20,21-époxy-20-éthyl-1-heneicosyl-diphosphate; 21,22-époxy-21-méthyl-1-docosyl-diphosphate ; 21,22-époxy-21-méthyl-1-docosyl-diphosphate.

L'invention concerne en particulier les composés phosphoépoxydes répondant à la formule suivante: 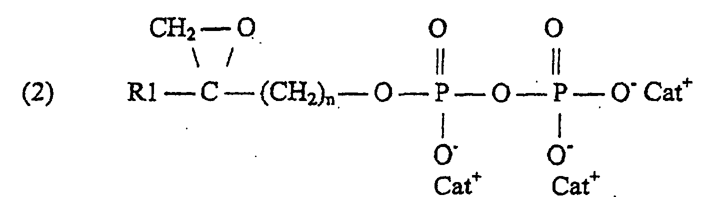 pour leur utilisation à titre de substances thérapeutiquement actives. Il est à noter que la publication M. MUEHLBACHER *et al.* "Isopentenyl-diphosphate isomerase: inactivation of the enzyme with active-site-directed irreversible inhibitors and transition-state analogs", Biochemistry, vol. 27, n° 19, p 7315-7328 (1988), décrit déjà un composé selon la formule (2) dans lequel R1 est CH3 et n = 2, ainsi que son effet *in vitro* en tant qu'inhibiteur de l'enzyme isopentènyl pyrophosphate isomèrase purifiée à partir de la moisissure *Claviceps purpurea.* Ce document n'enseigne aucune application thérapeutique de ce composé.

L'invention concerne aussi des nouveaux composés comprenant au moins un groupement phosphoépoxyde de formule :

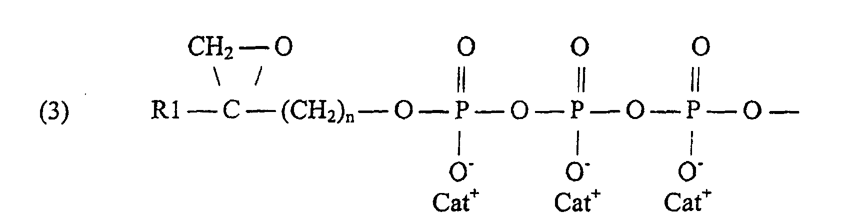

Parmi les nouveaux composés selon l'invention répondant à la formule (3), on peut citer les composés phosphoépoxydes de formule : 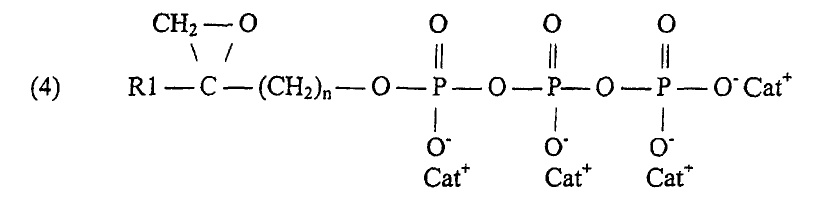 et les composés phosphoépoxydes de formule : 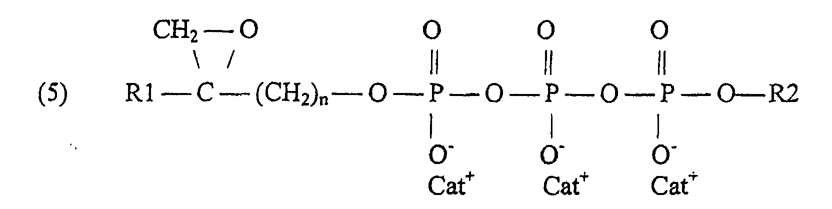 où R2 est un substituant organique ou minéral choisi dans le groupe formé :
- des substituants qui n'empêchent pas la formation de la fonction halohydrine 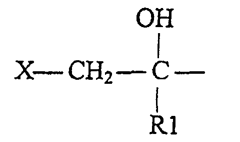 à partir de la fonction alcène 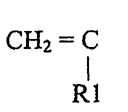 et de l'halogène X₂ en présence d'eau ;
- des substituants pour lesquels il existe un composé R2-O-Y non réactif sur la fonction halohydrine du composé de formule : 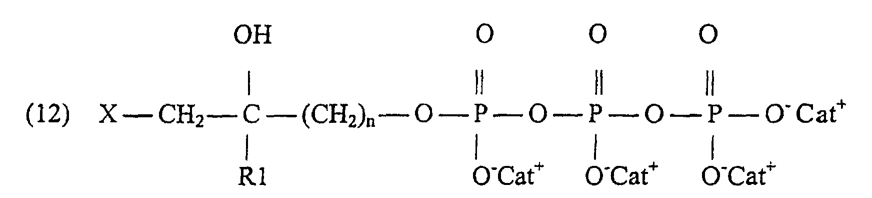 et choisi pour que R2-O-Y puisse réagir sur le phosphate terminal de ce composé (12) pour obtenir le composé de formule (15) : 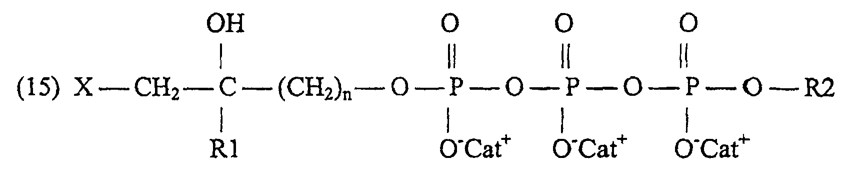
- et des substituants pour lesquels il existe un composé R2-O-PPP, où PPP symbolise le groupement triphosphate.

Avantageusement, lesdits composés selon l'invention sont caractérisés en ce que n = 2 et R1 est CH₃.

Les composés selon l'invention comprennent avantageusement en outre au moins un groupement choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des halohydrines, et des époxydes.

En particulier, l'invention s'étend aux composés phosphoépoxydes selon la formule (5) ci-dessus dans lesquels R2 est en outre choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des halohydrines, des phosphoépoxydes selon la formule (1), et des époxydes.

L'invention s'étend aussi aux composés dont la structure incorpore plusieurs groupements conformes à la formule (1), identiques ou différents, par exemple des monomères, polymères, oligomères ou dendrimères, ou plus généralement des molécules à plusieurs branches phosphatées conformes à la formule (1).

Il est à noter que les composés selon l'invention sont des esters (monoesters ou diesters) d'acide phosphorique (ce terme englobant les acides où le phosphore est au degré d'oxydation V, à savoir l'acide orthophosphorique, l'acide pyrophosphorique, l'acide métaphosphorique, l'acide triphosphorique, les autres acides polyphosphoriques).

L'invention s'étend à un procédé de fabrication des composés selon l'invention. Ce procédé est caractérisé en ce que :
- on prépare tout d'abord un composé intermédiaire comprenant au moins un groupement phosphohalohydrine de formule : 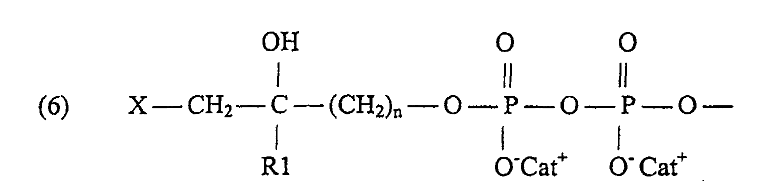
où X est un halogène choisi parmi l'iode, le brome et le chlore,
- on fait réagir le composé intermédiaire avec un milieu générateur d'hydroxydes pour transformer les fonctions halohydrines du composé intermédiaire en fonctions époxydes.

Avantageusement et selon l'invention pour préparer ledit composé intermédiaire, on fait réagir l'halogène X₂ en présence d'eau avec un composé de départ comprenant au moins un groupement alcène phosphaté de formule :

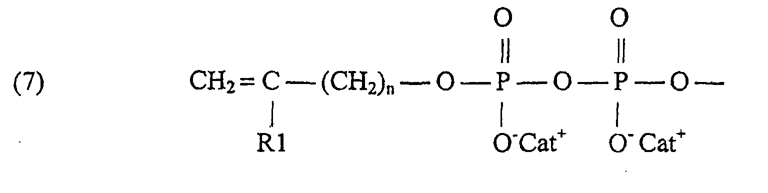

Avantageusement et selon l'invention, on fait réagir un sel formé dudit composé de départ en milieu aqueux ou hydroalcoolique, à pH neutre, à une température inférieure à 30°C, par mélange avec une solution aqueuse de l'halogène X₂. Avantageusement et selon l'invention, on effectue la réaction sous pression atmosphérique à une température comprise entre 0°C et 25°C.

Les composés de départ peuvent être eux-mêmes obtenus à partir de l'alcool :

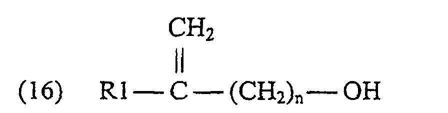

Avantageusement et selon l'invention, le composé de départ est un sel de formule :

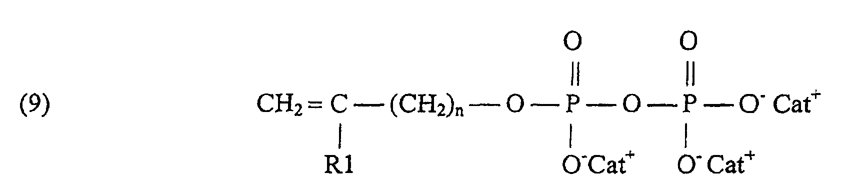

On obtient alors le composé intermédiaire pyrophosphohalohydrine selon la formule :

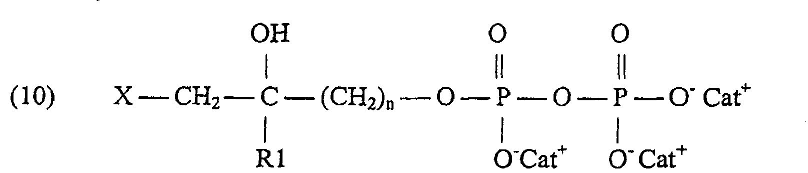

Un exemple de schéma réactionnel complet d'obtention du composé intermédiaire (10) à partir de l'alcool (16) est le suivant : 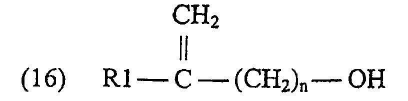 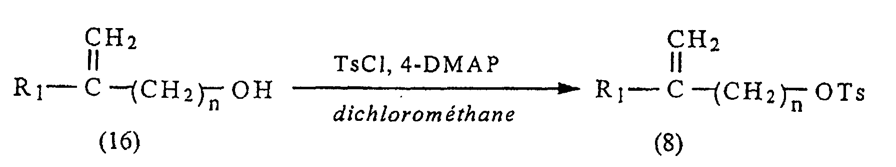 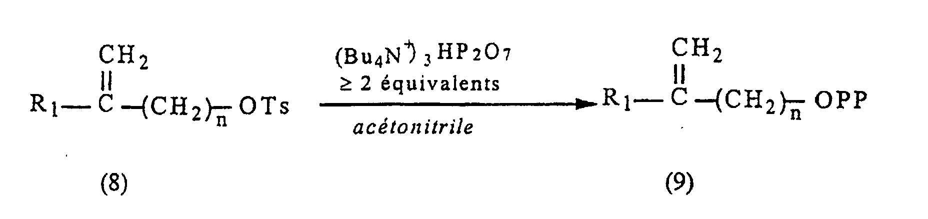 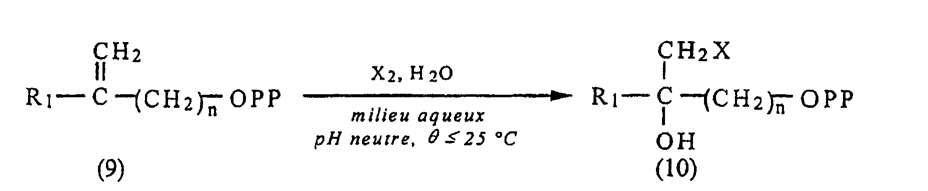
où TsCl est le chlorure de tosyle,
4-DMAP est le 4-diméthylaminopyridine,
Bu₄ N+ est le tétrabutylammonium,
(Bu₄N+)₃HP₂O₇ est le tris (tétra n-butylammonium) hydrogènopyrophosphate,
PP symbolise le groupement pyrophosphate.

Les réactions permettant d'obtenir le composé (9) à partir de l'alcool (16) peuvent être effectuées comme décrit par : DAVISSON V.J. *et al.* "Phosphorylation of Isoprenoid Alcohols" J. Org. Chem 1986, 51,4768-4779, et DAVISSON V.J. *et al.* "Synthesis of Allylic and Homoallylic Isoprenoid Pyrophosphates" Methods in Enzymology, 1984, 110, 130-144.

A partir du composé intermédiaire (10), on obtient le composé selon l'invention de formule (2) selon le schéma suivant :

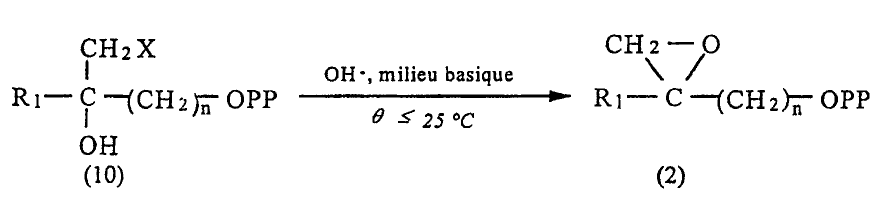

Avantageusement et selon l'invention, le composé de départ est un sel de formule :

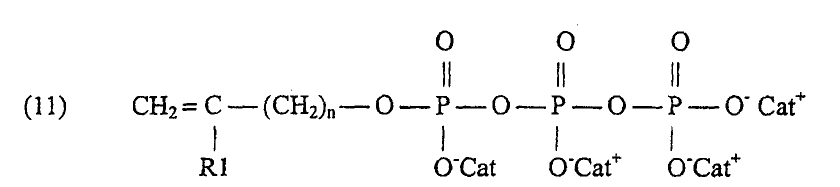

On obtient alors le composé intermédiaire triphosphohalohydrine selon la formule:

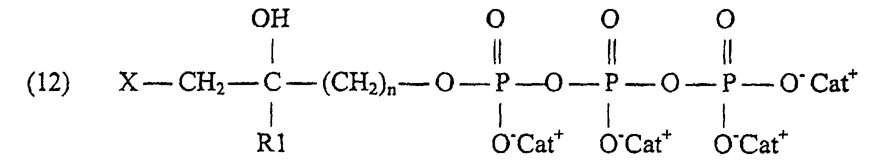

On obtient ensuite le composé triphosphoépoxyde (4) selon l'invention.

Un exemple de schéma réactionnel complet d'obtention du composé (4) selon l'invention à partir de l'alcool (16) est le suivant : 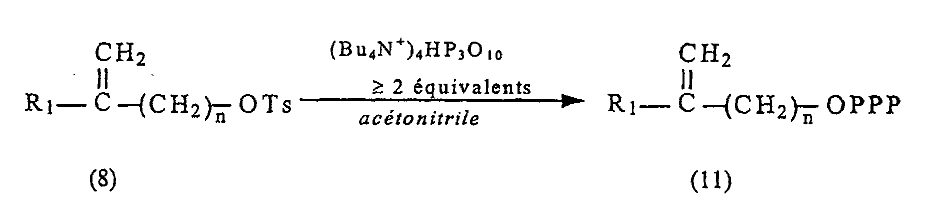 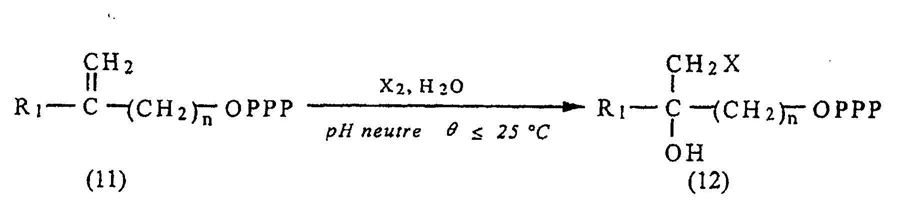 où PPP est le groupement triphosphate,
(Bu₄N⁺)₄HP₃O₁₀ est le tétrakis (tétra n-butylammonium) hydrogènotriphosphate. Le composé (8) est obtenu à partir de l'alcool (16) comme indiqué précédemment.

A partir du composé intermédiaire (12), on obtient le composé triphosphoépoxyde selon l'invention de formule (4) selon le schéma suivant :

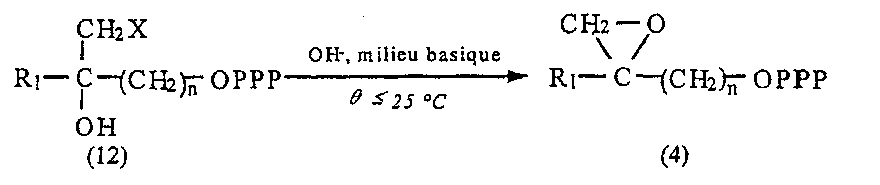

Pour préparer un composé phosphoépoxyde selon l'invention conforme à la formule (5), plusieurs variantes sont possibles.

Dans une première variante, on prépare tout d'abord un composé intermédiaire phosphohalohydrine de formule :

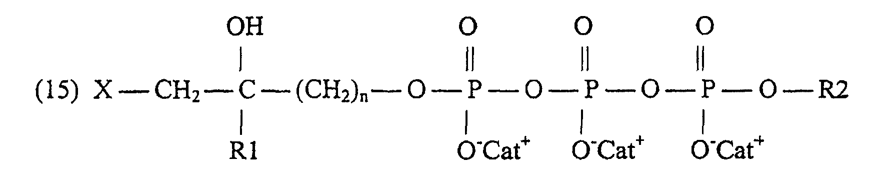

Ainsi, on obtient le composé (5) selon la réaction suivante :

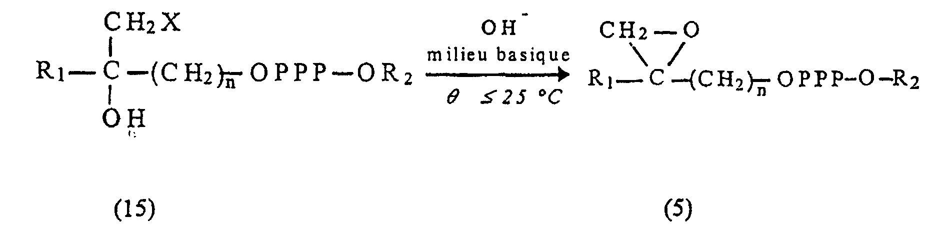

Pour ce faire, on peut effectuer le procédé tel que mentionné ci-dessus (réaction de X₂ en présence d'eau sur une fonction alcène phosphatée) en prenant, à titre de composé de départ, un sel de formule : 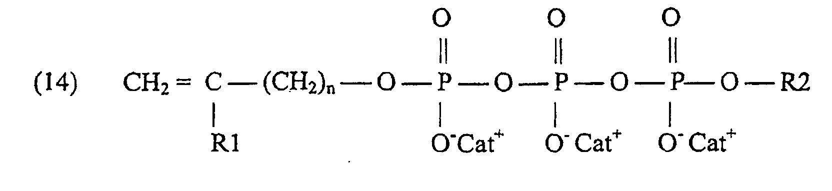 où R2 est un substituant organique ou minéral adapté pour ne pas empêcher la formation de la fonction halohydrine 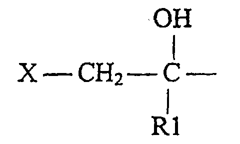 à partir de la fonction alcène 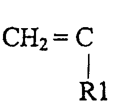 et de l'halogène X₂ en présence d'eau.

Le composé de départ (14) peut lui-même être préparé selon l'un des schémas réactionnels suivants : 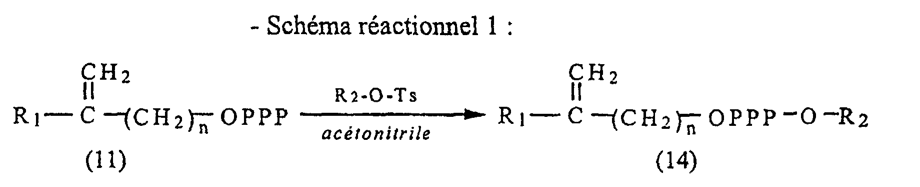 où Ts est le tosyle.

Le composé (11) peut être obtenu comme indiqué précédemment à partir de l'alcool (16) et du composé intermédiaire (8). La réaction permettant d'obtenir le composé (14) à partir du composé (11) peut être effectuée dans des conditions semblables à celles décrites dans les publications DAVISSON V.J. *et al.* Ce schéma peut être utilisé lorsque R2-O-Ts est commercialement disponible.

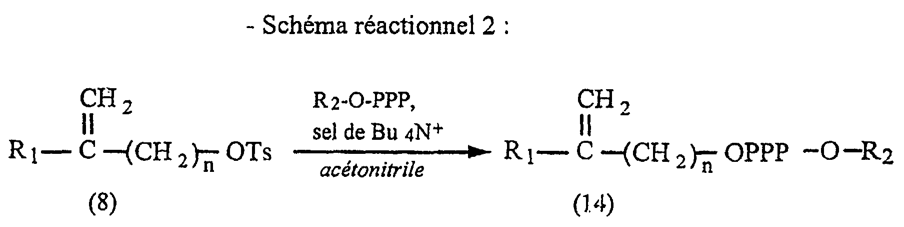

Le composé intermédiaire (8) peut être obtenu comme indiqué précédemment à partir de l'alcool (16). La réaction permettant d'obtenir le composé (14) à partir du composé (8) peut être effectuée dans des conditions semblables à celles décrites dans les publications DAVISSON V.J. *et al.* Ce schéma peut être utilisé lorsque R2-O-PPP est commercialement disponible. 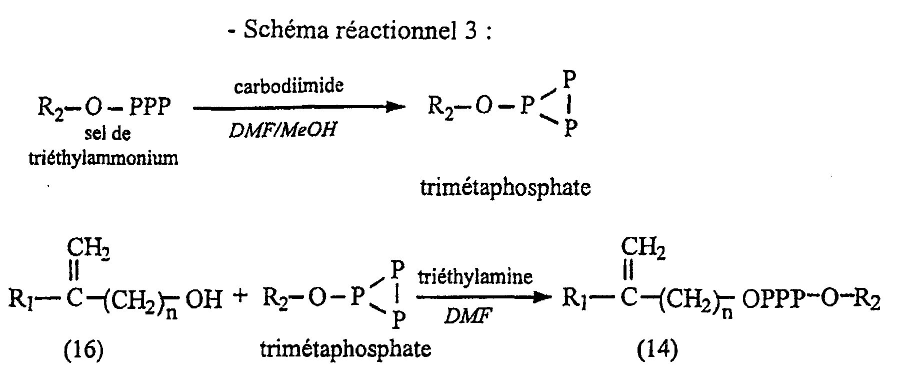 où DMF est le diméthylformamide,
MeOH est le méthanol.

Ce schéma réactionnel 3 peut être mis en oeuvre dans des conditions similaires à celles décrites dans D.G. KNORRE *et al* "Général method for the synthesis of ATP gamma derivatives" Febs LETTERS, 1976, 70, 105-108.

Ce schéma réactionnel 3 n'est pas utilisable lorsque R2 comporte une fonction réactive au carbodiimide (carboxylate, triphosphate...). Elle est par contre avantageuse lorsque R2-O-PPP est commercialement disponible.

Dans le cas particulier où R2- est lui-même un groupement époxyde de formule : 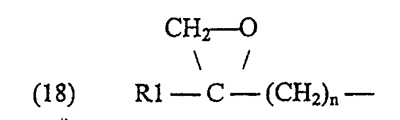 on peut utiliser le schéma réactionnel suivant :

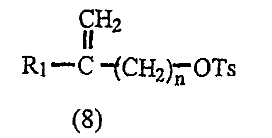

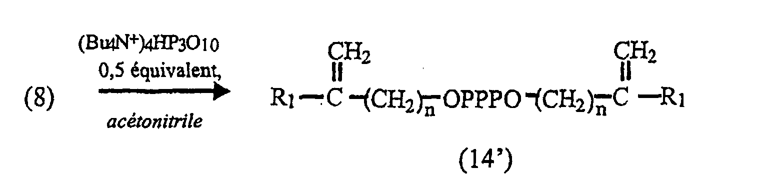

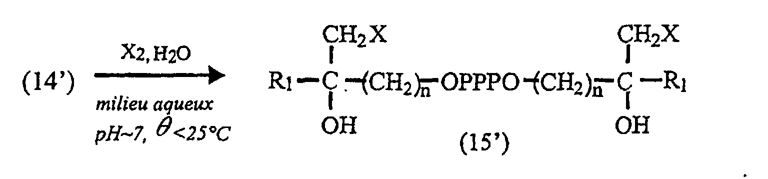

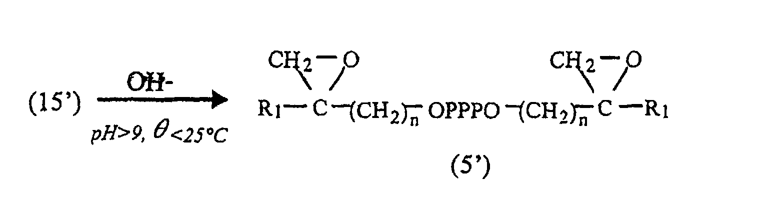

Le composé (5') est un cas particulier de composé (5) selon l'invention.

Il est à noter que dans toutes ces réactions, l'acétonitrile peut être remplacé par tout autre solvant dipolaire aprotique (diméthylformamide DMF, diméthylsulfoxyde DMSO...).

Il est à noter qu'à la place du composé intermédiaire (8) pour la préparation des composés (2), (4) et (5'), et dans le cas où n ≠ 2 on peut aussi utiliser le composé chlorure ou bromure correspondant de formule : 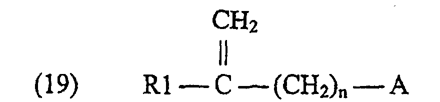 où A est le chlore ou le brome.

Les alcools (16) sont commercialement disponibles ou peuvent être aisément obtenus par une réaction de Grignard bien connue entre un organomagnésien d'alcényle et le formaldéhyde ou l'oxyde d'éthylène.

Dans une deuxième variante, pour préparer le composé intermédiaire (15), dans certains cas, on pourrait faire réagir le composé triphosphohalohydrine intermédiaire de formule (12), à partir d'un sel soluble en milieu organique tel qu'un sel de Bu₄N+, dans une étape ultérieure avec un composé R2-O-Y, où -O-Y est un groupement partant et R2 est un substituant organique ou minéral choisis pour que R2-O-Y puisse former, par réaction sur le composé (12), le composé intermédiaire de formule (15).

Pour pouvoir former ainsi le composé intermédiaire selon la formule (15), le composé R2-O-Y ne doit notamment pas être réactif sur la fonction halohydrine : 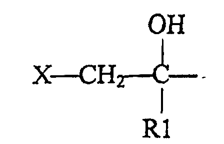 En outre, R2-O-Y doit réagir sur le phosphate terminal du composé (12) pour former le composé (15).

La réaction du composé de formule (12) sur R2-O-Y est une substitution nucléophile. Cette réaction est en particulier possible et avantageuse pour R2 choisi dans le groupe formé des alkyles et des alcènes. Y est choisi de telle sorte que R2-O-Y puisse donner le composé (15) par substitution nucléophile. Y est par exemple choisi parmi le tosyle, le brosyle et le triflyle.

Cette deuxième variante permet ensuite de préparer le composé phosphoépoxyde selon l'invention de formule (5) en faisant réagir le composé intermédiaire (15) en milieu aqueux basique pour transformer les fonctions halohydrine du composé intermédiaire (15) en fonctions époxydes comme indiqué ci-dessus.

Dans une troisième variante, on peut préparer le composé phosphoépoxyde selon l'invention, sans passer par le composé intermédiaire (15), à partir de l'alcool (16) selon le schéma réactionnel suivant :

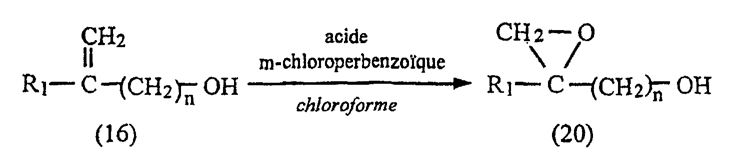

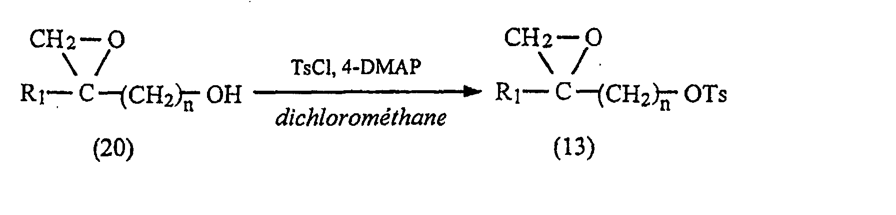

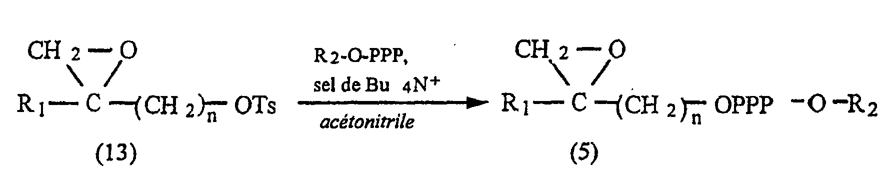

Cette troisième variante est notamment avantageuse lorsque R2-O-PPP est commercialement disponible ou lorsque la préparation du composé intermédiaire phosphohalohydrine (15) n'est pas possible.

La première étape de cette réaction consistant à transformer la fonction alcène en fonction époxyde, peut être effectuée comme indiqué par M. MUEHLBACHER *et al.* "Isopentenyl-diphosphate isomerase: inactivation of the enzyme with active-site-directed irréversible inhibitors and transition-state analogs", Biochemistry, vol. 27, n° 19, p 7315-7328 (1988).

Ce schéma réactionnel peut être aussi utilisé pour obtenir directement les composés monoesters (2) et (4) selon l'invention. Néanmoins, avec ces composés monoesters, la fabrication à partir des composés intermédiaires phosphohalohydrines est en général plus rapide, plus quantitative, et plus facile à mettre en oeuvre.

Dans une quatrième variante, on utilise le schéma réactionnel suivant :

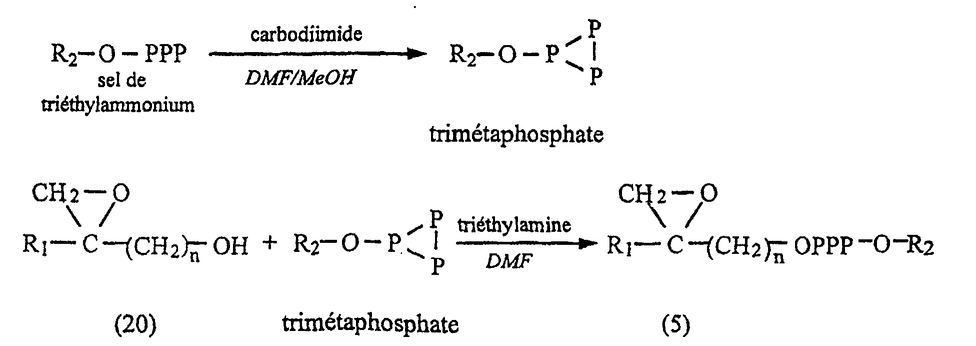

Cette réaction peut être effectuée dans les conditions décrites par D.G. KNORRE *et al* "General method for the synthesis of ATP gamma derivatives" Febs letters, 1976, 70, 105-108.

Ainsi, un composé selon l'invention peut être bifonctionnel ou multifonctionnel. La(les) fonction(s) phosphoépoxyde(s) procure(nt) la propriété antigénique vis-à-vis des lymphocytes Tγ9δ2, et R2 ou les autres groupements fonctionnels du composé peuvent présenter d'autres propriétés, notamment thérapeutiques.

Dans le cas d'un composé selon l'invention ayant plusieurs groupements phosphoépoxydes conformes à la formule (1), il suffit soit de partir d'un composé de départ ayant le nombre correspondant de groupements alcènes phosphatés de formule (7) et la structure chimique correspondante (première variante), soit d'utiliser le composé de formule (12) et de le faire réagir avec un composé intermédiaire R2-O-Y ayant le nombre correspondant de fonctions -O-Y (deuxième variante), soit d'utiliser un composé R2-O-PPP incorporant déjà d'autres fonctions époxydes (troisième et quatrième variantes).

L'invention concerne aussi en particulier les nouveaux composés β-esters de phosphoépoxydes de formule : 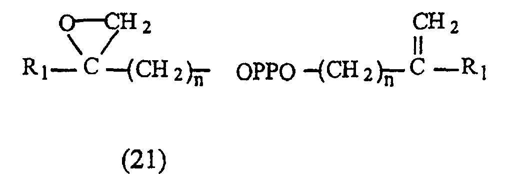 où
R1 est choisi parmi ―CH₃ et ―CH₂―CH₃,
n est un nombre entier compris entre 2 et 20.

Ces composés peuvent être obtenus selon le procédé suivant :

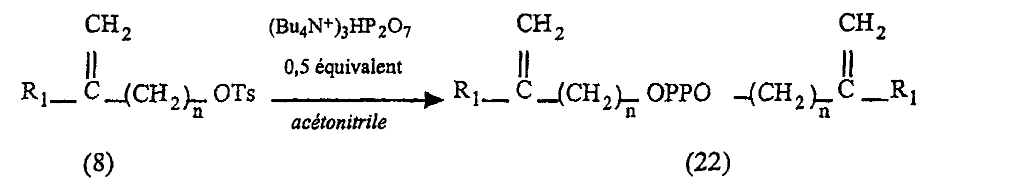

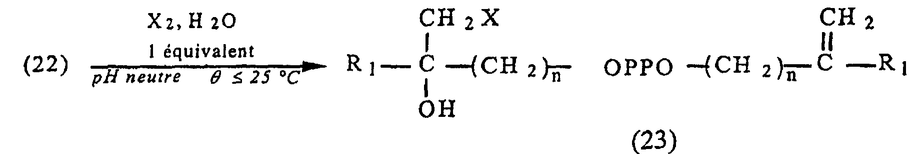

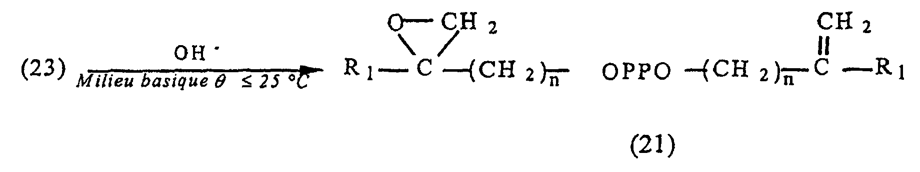

L'invention s'étend également aux utilisations des composés selon l'invention à titre d'activateur des lymphocytes Tγ9δ2 des primates, notamment à titre d'activateur de la prolifération et/ou de l'activité cytotoxique et/ou de la production de substance(s) médiatrice(s) des lymphocytes Tγ9δ2 des primates à récepteurs TCR comprenant les régions variables Vγ9 et Vδ2.

L'invention s'étend aussi aux applications des composés selon l'invention pour le traitement de cellules sensibles aux lymphocytes Tγ9δ2 des primates, dans un milieu naturel ou artificiel pouvant contenir des lymphocytes Tγ9δ2, dans lequel lesdites cellules peuvent être mises en contact avec ces lymphocytes Tγ9δ2, et qui est compatible avec les composés selon l'invention (c'est-à-dire n'est pas susceptible d'en provoquer la dégradation au moins dans certaines conditions du traitement).

Par "cellule sensible aux lymphocytes Tγ9δ2", on entend toute cellule sujette à l'activité effectrice induite des lymphocytes Tγ9δ2 : mort cellulaire (destruction cellulaire par les lymphocytes Tγ9δ2); réception de cytokine relarguée par les lymphocytes Tγ9δ2 (TNF-α, INF-γ...); éventuellement prolifération cellulaire induite par les lymphocytes Tγ9δ2.

L'invention s'étend donc à un procédé d'activation des lymphocytes Tγ9δ2 -notamment à un procédé d'activation de la prolifération des lymphocytes Tγ9δ2 et/ou de l'activité cytotoxique des lymphocytes Tγ9δ2 et/ou de la production de substance(s) médiatrice(s) par les lymphocytes Tγ9δ2- dans lequel on met ces lymphocytes Tγ9δ2 au contact d'au moins un composé selon l'invention dans un milieu contenant des lymphocytes Tγ9δ2 compatible avec la croissance lymphocytaire T. Avantageusement et selon l'invention, on introduit dans le milieu une proportion d'interleukine -notamment d'interleukine 2 (IL2)-adaptée pour engendrer une croissance lymphocytaire dans le milieu. En effet, la présence du facteur de croissance lymphocytaire IL2 est indispensable pour obtenir la prolifération des lymphocytes T parmi lesquels seuls les lymphocytes Tγ9δ2 ont été activés par un composé selon l'invention. Ainsi, ce facteur de croissance doit être présent dans le milieu pour les applications où l'on recherche une prolifération des lymphocytes Tγ9δ2. Ce facteur de croissance lymphocytaire peut préexister à l'état naturel, ou être induit ou introduit dans le milieu, simultanément ou non à l'incorporation du composé selon l'invention, dans la même composition thérapeutique ou non. Néanmoins, pour certaines applications où une activation sans prolifération des lymphocytes Tγ9δ2 est recherchée (par exemple la cytotoxicité induite), la présence de ce facteur de croissance n'est pas utile.

Plus particulièrement, l'invention s'étend aux applications des composés selon l'invention à titre thérapeutique pour le traitement curatif ou préventif des pathologies produisant des cellules sensibles aux lymphocytes Tγ9δ2 des primates dans un milieu pouvant contenir ces lymphocytes Tγ9δ2 et dans lequel ces cellules peuvent être mises au contact des lymphocytes Tγ9δ2.

Avantageusement et selon l'invention, on utilise au moins un composé selon l'invention à une concentration dans le milieu qui procure une activation de la prolifération polyclonale des lymphocytes Tγ9δ2. Ce milieu peut être choisi parmi le sang humain, le sang d'un primate non humain, les extraits de sang humain, et les extraits de sang d'un primate non humain.

Ledit milieu peut être extracorporel, ledit procédé d'activation selon l'invention étant alors un traitement cellulaire extracorporel, pouvant notamment servir en laboratoire, par exemple pour l'étude des lymphocytes Tγ9δ2 ou de leurs propriétés, ou à des fins de diagnostic. L'invention s'étend aussi à une composition pour le diagnostic extracorporel (*ex vivo*) caractérisée en ce qu'elle comprend au moins un composé selon l'invention.

Ledit milieu peut être aussi intracorporel, l'activation des lymphocytes Tγ9δ2 ayant alors une utilité thérapeutique.

Plus particulièrement, ledit milieu est le sang périphérique d'un primate. L'invention s'étend donc en particulier à un procédé d'activation des lymphocytes Tγ9δ2 du sang périphérique d'un primate -notamment de l'homme- dans lequel on administre une quantité apte à activer les lymphocytes Tγ9δ2 d'au moins un composé selon l'invention. On administre donc au moins un composé selon l'invention par voie générale -notamment parentérale dans le sang périphérique-.

Ledit milieu peut aussi être un site cellulaire à traiter, et on administre au moins un composé selon l'invention directement au contact du site cellulaire à traiter (administration topique).

L'invention s'étend ainsi en particulier aux applications thérapeutiques des composés selon l'invention pour le traitement des pathologies des primates appartenant au groupe formé des cancers, des maladies infectieuses, notamment mycobactériennes (lèpre, tuberculose...) ; des parasitoses (paludisme...) ; des pathologies à syndrome d'immunodéficience (SIDA, ...). Selon l'invention, on administre une composition thérapeutique adaptée pour libérer dans le sang périphérique et/ou sur un site cellulaire à traiter une quantité d'au moins un composé selon l'invention apte à activer les lymphocytes Tγ9δ2. En effet, il a été démontré de façon générale dans l'art antérieur sus-cité qu'une composition ayant la propriété d'activer les lymphocytes Tγ9δ2 peut être avantageusement utilisée pour le traitement de ces pathologies.

De façon traditionnelle, dans tout le texte, les termes "thérapie" ou "thérapeutique" englobent non seulement les traitements curatifs ou les soins, mais également les traitements préventifs (prophylaxie) tels que la vaccination ainsi que le diagnostic intracorporel (administration à des fins de diagnostic). En effet, en permettant l'activation des lymphocytes Tγ9δ2, l'invention permet des traitements d'immunostimulation pouvant être avantageux aussi bien à titre prophylactique en empêchant le développement de cellules pathogènes sensibles aux lymphocytes Tγ9δ2, qu'à titre curatif en induisant la destruction de cellules pathogènes sensibles aux lymphocytes Tγ9δ2.

L'invention s'étend ainsi à une composition thérapeutique comprenant au moins un composé selon l'invention. Plus particulièrement, l'invention concerne une composition thérapeutique comprenant une quantité apte à être administrée à un primate -notamment au contact du sang périphérique ou par voie topique d'au moins un composé selon l'invention -notamment pour le traitement préventif ou curatif des pathologies sus-citées-. Une composition selon l'invention peut être une composition immunostimulante, ou un vaccin, les composés selon l'invention étant des antigènes activant les lymphocytes Tγ9δ2.

Avantageusement et selon l'invention, la composition thérapeutique est caractérisée en ce qu'elle comprend en outre une proportion d'interleukine -notamment d'interleukine 2- adaptée pour engendrer une croissance lymphocytaire dans le milieu où elle est destinée à être administrée.

Une composition thérapeutique selon l'invention peut être préparée sous une forme galénique apte à être administrée par voie générale, notamment par voie parentérale directement dans le sang périphérique d'un primate, avec au moins un composé selon l'invention en quantité adaptée pour activer les lymphocytes Tγ9δ2 et un ou plusieurs excipient(s) approprié(s). Compte tenu de la très faible valeur de la concentration active des composés selon l'invention (de l'ordre de 1 à 100 nM), une telle administration est envisageable sans risque de toxicité.

Une composition thérapeutique selon l'invention peut aussi être préparée sous une forme galénique appropriée pour son administration topique, directement au contact des cellules sensibles aux lymphocytes Tγ9δ2.

La forme galénique d'une composition thérapeutique selon l'invention est réalisée selon la voie d'administration choisie, par les techniques traditionnelles de formulation galénique. La quantité et la concentration de composé(s) selon l'invention, et la posologie sont déterminées par référence aux traitements chimiothérapeutiques connus des maladies à traiter, compte tenu de la bioactivité des composés selon l'invention vis-à-vis des lymphocytes Tγ9δ2, de l'individu à traiter, de la maladie concernée et des effets biologiques recherchés.

Avantageusement et selon l'invention, pour un composé bioactif à une concentration comprise entre 10nM et 100nM on administre par voie générale une quantité de composé(s) selon l'invention comprise entre 1µg et 1000µg -notamment entre 10µg et 100µg- par kilogramme de poids du patient.

Par ailleurs, il a été démontré *in vitro* que les composés selon l'invention ne présentent aucune toxicité générale, même pour des concentrations pouvant aller jusqu'à 100µM, soit de l'ordre de 10⁴ fois la concentration bioactive. En outre, on sait que la catégorie biochimique de molécules à laquelle les composés selon l'invention appartiennent (phosphoesters) constitue une famille de composés métabolites rencontrés dans toute cellule vivante. Les composés selon l'invention ne présentent donc pas d'autres effets toxiques que ceux induits par leur bioactivité sur les lymphocytes Tγ9δ2.

En outre, certains composés selon l'invention présentent un poids moléculaire suffisamment faible (notamment inférieur à 500) pour être compatible avec leur élimination par voie rénale et urinaire.

Un exemple de formulation de composition thérapeutique injectable selon l'invention pour un primate de 1kg est le suivant : 50µg de 3,4-époxy-3-méthyl-1-butyl-diphosphate (Epox-PP) dilués dans 0,5ml de tampon phosphate stérile à pH 7 amenés à 37°C.

On administre ainsi 50µg d'Epox-PP (composé de formule (2)) pour 1kg d'animal, correspondant à une concentration dans le sang circulant adaptée pour être supérieure à la concentration bioactive de l'Epox-PP (une concentration de 100nM d'Epox-PP correspondant à environ 50ng/ml).

Il est à noter que les excipients ou autres additifs pharmaceutiquement acceptables traditionnellement utilisés, sont chimiquement compatibles avec les composés selon l'invention.

Une composition thérapeutique selon l'invention peut aussi avantageusement comprendre un ou plusieurs autre(s) principe(s) actif(s), notamment pour procurer un effet synergique. En particulier, un composé selon l'invention peut faire office d'adjuvant de vaccin. La composition thérapeutique vaccinante selon l'invention est alors formée d'une composition vaccinante connue à laquelle on rajoute une quantité de composé(s) selon l'invention apte à activer les lymphocytes Tγ9δ2 qui non seulement pourront exercer directement leur activité anti-infectieuse, mais aussi activer les lymphocytes T effecteurs de la réponse vaccinale traditionnelle.

Une composition thérapeutique selon l'invention peut aussi incorporer elle-même des lymphocytes Tγ9δ2 de primates en culture dans un milieu compatible avec la croissance lymphocytaire T. Elle peut alors servir au traitement des primates, ou plus généralement des animaux vertébrés avec lesquels l'administration des lymphocytes Tγ9δ2 de primates peut être effectuée dans des conditions de compatibilité immunitaire vis-à-vis desdits lymphocytes Tγ9δ2 de primates. Une telle composition selon l'invention peut être administrée par voie générale, ou même par voie topique, au contact des cellules cibles pathogènes, sensibles auxdits lymphocytes Tγ9δ2 de primates.

L'invention s'étend aussi à l'utilisation d'au moins un composé selon l'invention pour la fabrication d'une composition thérapeutique selon l'invention, Plus particulièrement, l'invention porte sur l'utilisation d'au moins un composé selon l'invention pour la fabrication d'une composition thérapeutique destinée au traitement préventif ou curatif d'une pathologie de l'homme ou de l'animal vertébré produisant des cellules sensibles aux lymphocytes Tγ9δ2 des primates -notamment une pathologie sélectionnée dans le groupe formé des cancers, des maladies infectieuses, des parasitoses et des pathologies à syndrome d'immunodéficience-. A ce titre, l'invention s'étend aussi à l'utilisation d'au moins un composé selon l'invention pour la fabrication d'une composition thérapeutique destinée à être administrée -notamment au contact du sang périphérique ou par voie topique- à un primate -notamment à l'homme- pour le traitement préventif ou curatif d'une pathologie telle que mentionnée ci-dessus.

L'invention s'étend aussi à un procédé de fabrication d'une composition -notamment une composition thérapeutique- selon l'invention ayant la propriété d'activer les lymphocytes Tγ9δ2, dans lequel on utilise au moins un composé selon l'invention.

L'invention porte aussi sur un procédé de fabrication d'une composition thérapeutique destinée au traitement préventif ou curatif d'une pathologie de l'homme ou de l'animal vertébré produisant des cellules pathogènes sensibles aux lymphocytes Tγ9δ2 de primates, dans lequel on utilise au moins un composé selon l'invention. L'invention porte en particulier sur un procédé de fabrication d'une composition thérapeutique destinée à être administrée - notamment au contact du sang périphérique ou par voie topique- à un primate pour le traitement préventif ou curatif d'une pathologie produisant des cellules sensibles aux lymphocytes Tγ9δ2 -notamment une pathologie appartenant au groupe mentionné ci-dessus-, dans lequel on utilise au moins un composé selon l'invention.

Avantageusement et selon l'invention, dans un procédé de fabrication selon l'invention, on met au moins un composé selon l'invention au contact d'un milieu contenant des lymphocytes Tγ9δ2 de primates, et compatible avec la croissance lymphocytaire T, en une quantité adaptée pour activer ces lymphocytes Tγ9δ2 dans ce milieu. Avantageusement et selon l'invention, ledit milieu comprend une substance choisie parmi le sang des primates et les extraits de sang dès primates. On obtient alors une composition thérapeutique contenant des lymphocytes Tγ9δ2 activés, permettant de réaliser une approche thérapeutique cellulaire.

Il est à noter que les composés selon l'invention sont époxydiques et ne correspondent pas aux phosphoantigènes naturels, notamment aux molécules dites Tubag1, Tubag2, Tubag3 et Tubag4 obtenues comme décrit par WO 95/20673. En effet, on démontre par exemple que ces phosphoantigènes naturels sont dégradés par l'eau de brome ou par traitement au borohydrure de sodium en milieu aqueux basique, alors que les composés selon l'invention ne sont pas sensibles à ces réactifs. Les composés selon l'invention ne sont donc pas des antigènes naturels, mais sont des antigènes synthétiques activateurs des lymphocytes Tγ9δ2 à des concentrations du même ordre, et avec une efficacité semblable voire même supérieure à celle des antigènes naturels.

Il est aussi à noter que, contrairement à l'état de la technique tel qu'illustré par US-5639653 qui considérait que la présence d'un groupe alkyle ou alcène était indispensable pour activer les lymphocytes Tγ9δ2 humains, les inventeurs ont constaté qu'en détruisant la liaison alcène en lui substituant un groupe époxyde, une activation des lymphocytes Tγ9δ2 extrêmement forte et à très faible concentration est obtenue. En particulier, on constate que l'effet peut même dépasser celui des phosphoantigènes d'origine naturelle.

D'autres caractéristiques, buts et avantages de l'invention apparaissent à la lecture des exemples qui suivent donnés à titre non limitatifs uniquement à des fins de compréhension, ainsi que des figures dans lesquelles :
- la figure 1 est un graphe représentant des résultats obtenus dans l'exemple 5,
- la figure 2 est un graphe représentant des résultats obtenus dans l'exemple 6.

### EXEMPLE 1 : Fabrication du 3,4-époxy-3-méthyl-1-butyl-diphosphate (Epox-PP)

### Préparation du 3-méthyl-3-butène-1-yl -tosylate (isopentènyl tosylate) :

Dans un réacteur en verre équipé pour la manipulation sous atmosphère inerte et soigneusement séché, sont introduits sous agitation magnétique (2,32 mmoles - 442mg) de chlorure de tosyle et (2,55 mmoles - 312 mg) de 4-(N,N-diméthylamino)pyridine dans 5 ml de dichlorométhane anhydre. A ce mélange, on ajoute lentement à l'aide d'une seringue et par l'intermédiaire d'un septum (2,32 mmoles - 200 mg) d'isopentènol en solution dans environ 1ml de dichlorométhane. La réaction est suivie par chromatographie sur couche mince de silice (gel de silice 60 F-254 - éluant : pentane/acétate d'éthyle 85/15 v/v - R_{f} (produit) = 0,4 et R_{f} (TsCl) = 0,5). Après environ 3 heures d'agitation sous atmosphère d'azote on dilue le mélange réactionnel dans un grand volume d'hexane (environ 100ml) ce qui entraîne la formation immédiate d'un précipité blanc. Le mélange est ensuite filtré et le filtrat concentré par évaporation sous pression réduite. La solution est diluée avec du diéthyl éther et filtrée à nouveau. Après évaporation du solvant, on obtient une huile jaunâtre. Le produit est purifié par chromatographie sur colonne préparatrice de silice (gel de silice 60 - éluant : pentane/acétate d'éthyle 85/15).(1,98 mmoles - 475 mg) de 3-méthyl-3-butène-1-yl -tosylate (85 % en rendement isolé) sont ainsi obtenus. Le composé (huile incolore) est stocké à + 4°C en milieu anhydre.

### Préparation du tris(tetra-n-butylammonium) hydrogènopyrophosphate :

(4,5 mmoles - 1g) de sel de disodium dihydrogènopyrophosphate (Na₂H₂P₂O₇) sont dissous dans 10 ml d'eau déionisée froide préalablement ajustée à pH 9 par une solution d'ammoniaque 10mM. La solution est passée sur une colonne contenant (19 milliéquivalents - 4 g) de résine cationique DOWEX® 50-WX8 -200 (forme H⁺). La solution acide est éluée avec 15-20 ml d'eau déionisée froide à pH 9. La solution collectée est immédiatement titrée à pH 7,3 par une solution aqueuse d'hydroxyde de tétra-n-butylammonium (Bu₄NOH) à 40%. Après lyophilisation on obtient 4 g de sel de tétra-n-butylammonium sous la forme d'un solide blanc hygroscopique. Le sel est dissout dans 10 ml d'acétonitrile anhydre. La solution est ensuite filtrée puis séchée par évaporations successives du solvant sous pression réduite. On obtient ainsi une solution de tris(tetra-n-butylammonium) hydrogènopyrophosphate avec une pureté égale à 98 % (résultat déduit de l'analyse par chromatographie ionique - HPAEC). Le volume est ajusté afin d'obtenir une concentration en sel comprise entre 0,5 et 1M. La solution est stockée à -20 °C en milieu anhydre.

### Préparation du 3-méthyl-3-butène-1-yl -diphosphate (isopentènyl pyrophosphate) :

Dans un réacteur en verre soigneusement séché, on introduit sous atmosphère d'azote 2,5 ml d'une solution de tris(tétra-n-butylammonium) hydrogènopyrophosphate à 0,7 M (1,75 mmoles) dans l'acétonitrile anhydre. Le réacteur est refroidi par un bain de glace puis on ajoute sous agitation magnétique et à l'aide d'une seringue (0,70 mmoles - 168 mg) de 3-méthyl-3-butène-1-yl -tosylate en solution dans un minimum d'acétonitrile (0,5 - 1M). Après introduction du tosylate, le bain de glace est retiré puis la réaction est laissée sous agitation à température ambiante. L'avancement de la réaction est alors suivi par chromatographie ionique (HPAEC). Après environ 3 heures, le solvant est évaporé sous pression réduite et le milieu réactionnel redissout dans 3 ml d'un mélange eau /2-propanol 98/2 (v/v). La solution est passée sur une colonne contenant (19 milliéquivalents - 4 g) de résine cationique DOWEX® 50-WX8 -200 (forme NH₄ ⁺) puis éluée avec 10 ml du mélange eau (pH 9)/2-propanol 98/2 (v/v). Après lyophilisation, on recueille un solide blanc contenant le produit brut.

### Purification :

Le pyrophosphate et les traces de monophosphate d'ammonium sont séparés du milieu par co-précipitation en présence d'hydrogénocarbonate d'ammonium. On dissout le produit brut obtenu à l'étape précédente dans 4 ml d'hydrogénocarbonate d'ammonium 0,1 M que l'on transfère dans un tube à centrifugation de 25 ml. On traite alors la solution avec 10 ml d'un mélange acétonitrile/2-propanol 1/1 (v/v) en agitant vigoureusement le mélange (vortex) pendant quelques minutes jusqu'à formation d'un précipité blanc. Le tube est ensuite centrifugé à 2000 tr/min à 10 °C pendant 5min. Le surnageant, dans lequel sont extraits les sels organiques, est conservé à +4°C. La procédure est renouvelée en redissolvant le précipité dans 3ml d'hydrogénocarbonate d'ammonium 0,1 M auxquels on ajoute 7 ml du mélange acétonitrile/2-propanol. Les deux surnageants sont regroupés et le solvant évaporé sous vide. On obtient un liquide huileux que l'on conserve à +4°C.

Le tosylate d'ammonium est séparé du milieu réactionnel par extraction avec le solvant chloroforme/méthanol 1/1 (v/v). Le liquide huileux de l'étape précédente est dissout dans 4 ml d'eau à pH 9 et traité avec 1ml de ce solvant par une procédure classique d'extraction répétée 3 fois. On élimine ensuite de la phase aqueuse les traces de solvant par évaporation sous pression réduite à 30 °C. On obtient sur la base de l'analyse par chromatographie ionique (HPAEC) un rendement de 83 % en 3-méthyl-3-butène-1-yl -diphosphate (0,58 mmoles - 172 mg). La solution est stockée à -20 °C.

Le produit est purifié ultérieurement selon les besoins par chromatographie d'échange d'anions sur cartouches Sep-Pak Accell Plus QMA (Waters®) de 360 mg à 10 grammes éluées successivement par des solutions aqueuses d'hydrogénocarbonate d'ammonium respectivement de 20 mM, 40 mM, 100 mM, puis 200 mM avec suivi chromatographique (HPAEC) des fractions éluées. Les fractions correspondant au produit purifié sont regroupées puis lyophilisées.

### Préparation du 3-(bromométhyl)-3-butanol-1-yl-diphosphate:

(0,34 mmole - 100 mg) d'isopentènyl-diphosphate (sel d'ammonium) en solution dans 2 ml d'eau déionisée de pH neutre sont traitées sous une hotte aspirante et à température ambiante par 1,9 ml (0,34 mrnoles) de brome en solution aqueuse saturée (0,18 M). L'eau de brome est ajoutée progressivement en agitant périodiquement jusqu'à décoloration de l'eau de brome. Dans le cas où le brome est ajouté en léger excès (coloration jaune persistante), la solution est transférée dans un ballon en verre puis placée quelques minutes sous pression réduite (évaporateur rotatif) à une température de 30 °C jusqu'à disparition de la coloration. La solution aqueuse est filtrée puis neutralisée par passage sur colonne de résine cationique DOWEX® 50-WX8-200 (forme NH₄ ⁺) éluée par deux volumes de colonne d'eau déionisée. On obtient quantitativement une solution de 3-(bromométhyl)-3-butanol-1-yl-diphosphate (0,33 mmoles - 130 mg) que est stockée à -20°C.

### Préparation du 3,4-époxy-3-méthyl-1-butyl-diphosphate :

On traite à température ambiante, 1ml de la solution aqueuse contenant (3,35 mg - 8,5 µmoles) de 3-(bromométhyl)-3-butanol-1-yl-diphosphate (sel d'ammonium) obtenue à l'étape précédente avec 1ml d'une solution molaire d'ammoniaque. La solution est maintenue sous agitation pendant quelques minutes puis lyophilisée pour éliminer l'ammoniaque. Le residu sec obtenu après lyophilisation (2,7 mg - 8,5 µmoles) est redissout dans 2 ml d'eau déionisée. Les ions bromures sont éliminés de la solution en utilisant un dispositif DIONEX® composé d'une cartouche OnGuard®-Ag fixée sur une cartouche OnGuard®-H. (capacité de 1,8 milliéquivalents). Les ions halogènures (bromures) sont retenus sélectivement par passage de la solution sur ce dispositif que l'on élue avec 1 ml d'eau déionisée. Pour la mise en oeuvre de tests biologiques les solutions aqueuses du produit sont stérilisées par filtration sur filtre de 0,2 µm et stockées à -20 °C. Dans le cas de tests réalisés *in vivo,* les solutions sont préalablement passées sur une colonne de résine cationique DOWEX® 50-WX8-200 (forme Na⁺) éluée par deux volumes de colonne d'eau déionisée.

### EXEMPLE 2 : Fabrication du 3,4-époxy-3-méthyl-1-butyl-triphosphate (Epox-PPP):

### Préparation du tétrakis(tétra-n-butylammonium) hydrogènotriphosphate :

(2,1 mmoles - 1g) de sel de pentasodium tripolyphosphate hexahydrate (Na₅P₃O₁₀.6H₂O) sont dissous dans 10 ml d'eau déionisée froide préalablement ajustée à pH 9 par une solution d'ammoniaque 10mM. La solution est passée sur une colonne contenant (21 milliéquivalents - 4,4 g) de résine cationique DOWEX® 50-WX8 (forme H⁺). La solution acide est éluée avec 20-25 ml d'eau déionisée froide à pH 9. La solution collectée est immédiatement titrée à pH 7,0 par une solution aqueuse d'hydroxyde de tétra-n-butylammonium (Bu₄NOH) à 40%. Après lyophilisation on obtient 2,5g de sel de tétra-n-butylammonium sous la forme d'un solide blanc hygroscopique. Le sel est dissout dans 10 ml d'acétonitrile anhydre. La solution est ensuite filtrée puis séchée par évaporations successives du solvant sous pression réduite. On obtient ainsi une solution de tetrakis(tetra-n-butylammonium) hydrogènotriphosphate avec une pureté égale à 95 % (résultat déduit de l'analyse par chromatographie ionique - HPAEC). Le volume est ajusté afin d'obtenir une concentration en sel comprise entre 0,5 et 1M. La solution est stockée à -20 °C en milieu anhydre.

### Préparation du 3-méthyl-3-butène-1-yl-triphosphate (isopentènyl-triphosphate):

En suivant la procédure décrite pour la préparation du 3-méthyl-3-butène-1-yl -diphosphate (exemple 1), on fait réagir sous atmosphère d'azote 2 mmoles d'une solution molaire de tetrakis(tetra-n-butylammonium) hydrogènotriphosphate avec (1 mmole - 240mg) de 3-méthyl-3-butène-1-yl-tosylate préparé selon l'exemple 1 dans 4 ml d'acétonitrile anhydre pendant 24 heures. En utilisant une procédure de purification par précipitation-extraction analogue à celle appliquée au 3-méthyl-3-butène-1-yl-diphosphate, on obtient sur la base de l'analyse par chromatographie ionique (HPAEC) un rendement de 74 % en 3-méthyl-3-butène-1-yl-triphosphate (0,74mmole - 292mg). Pour la préparation de phosphoépoxydes selon l'invention, dans le cadre de tests biologiques, on utilise une fraction du produit obtenu à ce stade que l'on purifie par HPAEC sur colonne IonPac® AS11 en cumulant plusieurs passages chromatographiques. On prépare de cette manière environ 2 ml d'une solution aqueuse millimolaire de pH neutre de 3-méthyl-3-butène-1-yl-triphosphate sous forme de sel d'ammonium.

### Préparation du 3-(bromométhyl)-3-butanol-1-yl-triphosphate :

500 nmoles (500 µl d'une solution millimolaire) d'isopentènyl triphosphate sont traitées à température ambiante par ajout de 500 nmoles de brome en solution aqueuse saturée (2,8 µl d'eau de brome à 180 mM). Après agitation du mélange et décoloration de l'eau de brome (quasi instantanée), le produit 3-(bromométhyl)-3-butanol-1-yl-triphosphate est généré quantitativement (0,5ml d'une solution millimolaire).

### Préparation du 3,4-époxy-3-méthyl-1-butyl-triphosphate :

La solution obtenue à l'étape précédente contenant 500 nmoles de 3-(bromométhyl)-3-butanol-1-yl-triphosphate est injectée en plusieurs fractions dans un système d'HPAEC Dionex® selon "High pH anion exchange chromatographic analysis of phosphorylated compounds : application to isolation and characterization of non peptide mycobacterial antigens", Y. Poquet *et al,* Anal. Biochem, 243 n° 1, 1996, p. 119-126. L'époxyde est formé quantitativement à chaque passage chromatographique et collecté en présence d'hydroxyde ou d'hydrogénocarbonate d'ammonium. Les fractions collectées sont lyophilisées. On prépare de cette manière environ 1 ml d'une solution aqueuse à 500 µM de 3,4-époxy-3-méthyl-1-butyl-triphosphate qui est traitée comme dans l'exemple 1 pour la mise en oeuvre de tests biologiques et/ou la réalisation d'essais *in vivo* et stockée à -20 °C.

### EXEMPLE 3 : Fabrication du α,γ di-(3,4-époxy-3-méthyl-1-butyl)-triphosphate (di EpoxTP) :

### Préparation du α,γ di-(3-méthyl-3-butène-1-yl)-triphosphate :

En suivant la procédure décrite pour la préparation du 3-méthyl-3-butène-1-yl -diphosphate (exemple 1), on fait réagir sous atmosphère d'azote 0,5 mmoles d'une solution molaire de tetrakis(tetra-n-butylammonium) hydrogènotriphosphate (préparé selon l'exemple 2) avec (1 mmole - 240 mg) de 3-méthyl-3-butène-1-yl-tosylate (préparé selon l'exemple 1) dans 4ml d'acétonitrile anhydre pendant 24 heures. En utilisant une procédure de purification par précipitation-extraction analogue à celle appliquée au 3-méthyl-3-butène-1-yl-diphosphate, on obtient sur la base de l'analyse par chromatographie ionique (HPAEC) un rendement de 81 % en α,γ di-(3-méthyl-3-butène-1-yl)-triphosphate (0,4 mmoles - 178 mg). Pour la préparation de phosphohalohydrines dans le cadre de tests biologiques, on utilise une fraction du produit obtenu à ce stade que l'on purifie par HPAEC sur colonne IonPac® AS11 en cumulant plusieurs passages chromatographiques. Avant chaque passage chromatographique et pour une meilleure isolation du produit, on effectue un traitement enzymatique à la phosphatase alcaline de la fraction à purifier pour dégrader l'isopentènyl triphosphate, qui est un sous produit de la réaction. On prépare de cette manière environ 1 ml d'une solution aqueuse millimolaire de pH neutre de α,γ di-(3-méthyl-3-butène-1-yl)-triphosphate sous forme de sel d'ammonium.

### Préparation du α,γ di-[3-(bromométhyl)-3-butanol-1-yl]-triphosphate :

250 nmoles (250 µl d'une solution millimolaire) de α,γ di-(3-méthyl-3-butène-1-yl)-triphosphate sont traitées à température ambiante par ajout de 250 nmoles de brome en solution aqueuse saturée (1,4 µl d'eau de brome à 180 mM). Après agitation du mélange et décoloration de l'eau de brome (quasi instantanée), le produit α,γ di-[3-(bromométhyl)-3-butanol-1-yl]-triphosphate est généré quantitativement (250 µl d'une solution millimolaire). Préparation du α,γ di-(3,4-époxy-3-méthyl-1-butyl)-triphosphate :

La solution obtenue à l'étape précédente contenant 250 nmoles de α,γ di-[3-(bromométhyl)-3-butanol-1-yl]-triphosphate est injectée en plusieurs fractions dans un système d'HPAEC Dionex® DX500 comme décrit dans l'exemple 2. On prépare de cette manière environ 0,5 ml d'une solution aqueuse à 500 µM de α,γ di-[3,4-époxy-3-méthyl-1-butyl]-triphosphate qui est traitée comme dans l'exemple 1 pour la mise en oeuvre de tests biologiques et/ou la réalisation d'essais *in vivo* et stockée à -20 °C.

### EXEMPLE 4 : Fabrication de l'uridine 5'-triphosphate γ-(3,4-époxy-3-méthyl-1-butyl) (Epox-UTP) :

### Préparation de l'uridine 5'-triphosphate γ-(3-méthyl-3-butène-1-yl):

Ce produit est préparé selon la procédure décrite par KNORRE D. C. et al. "Général Method for the synthesis of ATP Gamma-derivatives" Febs Letters, 1976, 70-1, 105-108, à partir de 40 µmoles d'uridine-5'-triphosphate (UTP) (sel de triéthylammonium) en présence d'un excès d'isopentènol. Pour la préparation de phosphépoxydes selon l'invention, dans le cadre de tests biologiques, une fraction du produit obtenu est purifié par HPAEC sur colonne IonPac ® AS11 en cumulant plusieurs passages chromatographiques. Avant chaque étape chromatographique et pour une meilleure isolation du produit, on effectue un traitement enzymatique à la phosphatase alcaline de la fraction à purifier pour dégrader les sous produits (UDP et UMP) et l'UTP n'ayant pas réagi. On prépare de cette manière environ 500 µl d'une solution aqueuse à 300 µM de pH neutre de l'uridine 5'-triphosphate γ-(3-méthyl-3-butène-1-yl) sous forme de sel d'ammonium.

### Préparation de l'uridine 5'-triphosphate γ-[3-(iodométhyl)-3-butanol-1-yl] :

75 nmoles (250 µl d'une solution 300 µM) de l'uridine 5'-triphosphate γ-3-méthyl-3-butène-1-yl sous forme de sel d'ammonium sont traitées en milieu aqueux de pH neutre par ajout de 108 µl d'eau iodée à 0,7 mM préparée selon l'exemple 2. La solution est laissée 20 minutes à température ambiante en effectuant périodiquement une agitation vigoureuse. Après décoloration de l'eau iodée, le produit l'uridine 5'-triphosphate γ-[3-(iodométhyl)-3-butanol-1-yl] est généré quantitativement (environ 360 µl d'une solution à 200 µM).

### Préparation de l'uridine 5'-triphosphate γ- (3,4-époxy-3-méthyl-1-butyl) :

La solution obtenue à l'étape précédente contenant 75 nmoles de l'uridine 5'-triphosphate γ-[3-(iodométhyl)-3-butanol-1-yl] est injectée en plusieurs fractions dans un système d'HPAEC Dionex® DX500 comme décrit dans l'exemple 2. On prépare de cette manière environ 0,5 ml d'une solution aqueuse à 150 µM de l'uridine 5'-triphosphate γ-(3,4-époxy-3-méthyl-1-butyl) qui est traitée comme dans l'exemple 1 par la mise en oeuvre de tests biologiques et/ou la réalisation d'essais *in vivo* et stockée à -20 °C.

### EXEMPLE 5 : Mesure de l'activité antigénique par stimulation de la prolifération des lymphocytes Tγ9δ2 en culture.

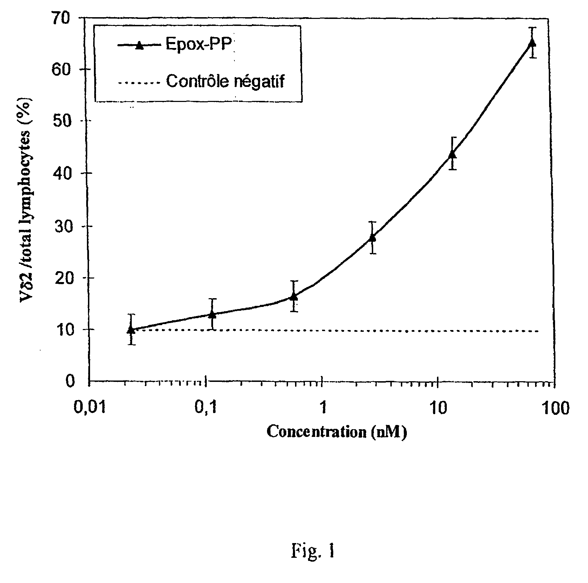

Dans une culture in vitro de 10⁶ lymphocytes T totaux dans 1ml, séparés à partir du sang d'un donneur humain sain adulte et contenant initialement de 1-5% de lymphocytes Tγ9δ2 en milieu de culture adéquat (RPMI 1640+10% de sérum humain inactivé et 50 U/ml d'interleukine 2 humaine (hIL-2), on rajoute 20 microlitres de solution aqueuse du composé selon l'invention à tester amené à la concentration finale spécifiée dans l'essai. Après 4 jours de culture, on rajoute par millilitre de milieu de culture 50 U d'hIL-2. Après huit jours les cellules sont énumérées, collectées, lavées par un tampon phosphate, et les cellules de type Tγ9δ2 sont révélées dans la culture par un marquage avec les réactifs commerciaux usuels (Anticorps monoclonaux fluorescéinés) et leur proportion déterminée par une analyse de cytométrie en flux. On prend en compte soit le changement de la proportion, soit l'augmentation numérique des cellules Tγ9δ2 dans des cultures en présence du composé selon l'invention par rapport à des cultures exemptes de composé selon l'invention. On représente le résultat de ces essais en traçant les courbes de ces valeurs (ordonnées figure 1) en fonction de la concentration en échelle logarithmique de composé selon l'invention mis en culture (abscisses figure 1).

La figure 1 représente les résultats obtenus avec le composé selon l'invention obtenu à l'exemple 1 (Epox-PP), la ligne en pointillés représentant un contrôle négatif (valeur obtenue en l'absence de composé selon l'invention).

Le tableau suivant illustre les valeurs de DE50, dose efficace à 50 % de l'effet maximal d'amplification lymphocytaire polyclonale obtenu comme indiqué ci-dessus, avec l'IPP (à titre comparatif), et avec différents composés selon l'invention.

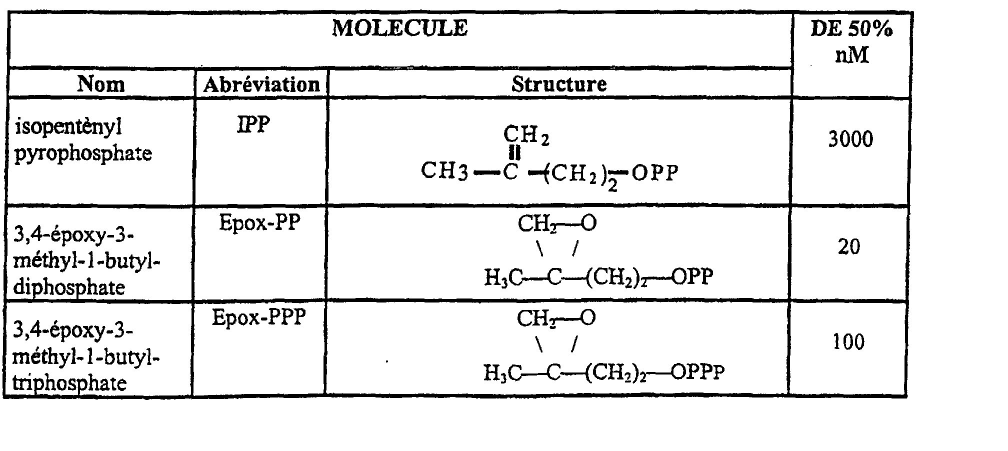

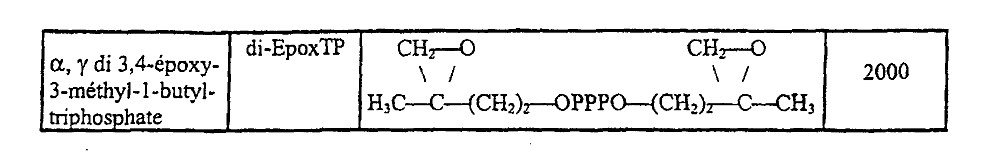

### EXEMPLE 6 : Mesure de l'activité antigénique par stimulation de la cytotoxicité induite :

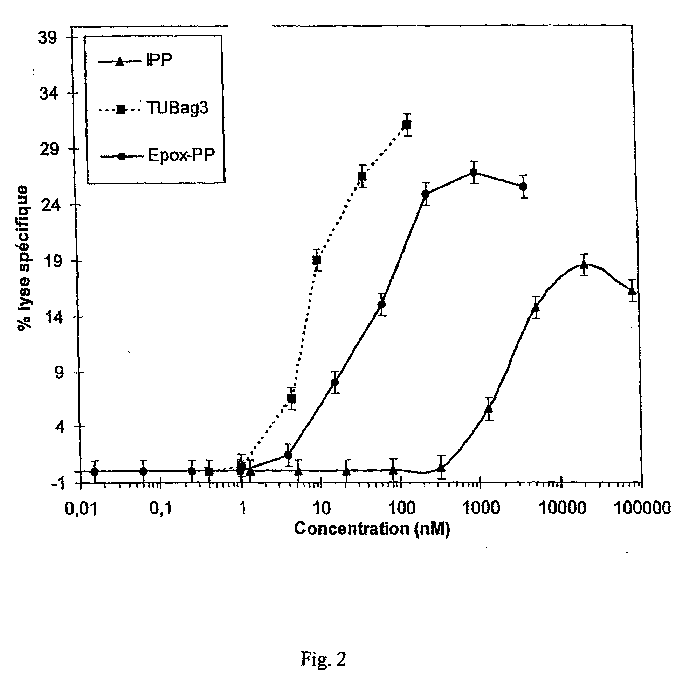

On compare l'activité cytotoxique spécifique d'un clone de lymphocyte Tγ9δ2 mesurée selon le test de cytotoxicité induite, cette activité étant simulée avec des concentrations décroissantes du phosphoantigène Tubag3 obtenu comme décrit par WO 95/20673 (courbe représentée par des carrés noirs figure 2), du composé Epox-PP selon l'invention obtenu à l'exemple 1 (courbe représentée par des ronds noirs figure 2), et de l'isopentènyl pyrophosphate (IPP, courbe représentée par des triangles noirs figure 2).

On constate que le composé selon l'invention de l'exemple 1 est actif à une concentration de l'ordre de 20 nM alors que l'IPP de l'art antérieur est actif à une concentration de l'ordre de 3 µM, soit 150 fois plus élevée.

### EXEMPLE 7 : Démonstration de la différence de structure entre un composé selon l'invention époxydique et les phosphoantigènes naturels Tubag mycobactériens.

On compare la bioactivité du composé selon l'invention Epox-PP (obtenu comme indiqué à l'exemple 1) à celle d'un stock de phosphoantigènes naturels Tubag obtenus comme décrit par WO 95/20673.

La bioactivité est mesurée par un test de cytotoxicité induite comme décrit à l'exemple 6 par le composé à tester dilué de 1:30 à partir de solutions des composés de 5 à 10µM.

Avant de mesurer la bioactivité, les composés sont soumis à un traitement chimique transitoire par contact avec l'un des réactifs suivants : NaIO₄ (5mM) ; NaBH₃CN (10mM, pH 7) ; KMnO₄ (1mM); eau de brome Br₂, H₂O (7mM), puis neutralisation du réactif.

Le tableau suivant donne les résultats obtenus par plusieurs essais indépendants réalisés dans chaque cas. + indique la détection d'une activité sur les lymphocytes Tγ9δ2, - indique qu'aucune activité n'a pu être détectée.

| Bioactivité après traitement chimique : | | | | | |
|---|---|---|---|---|---|
| REACTIF | aucun | Na IO₄ *(5mM)* | Na BH₃CN *(10mM pH7)* | KMnO₄ *(1mM)* | Br₂,H₂O *(7mM)* |
| Tubag | + | + | + | - | - |
| Epox-PP | + | + | + | + | + |

On constate que le traitement des phosphoantigènes naturels mycobactériens Tubag par le KMnO₄ dilué ou par l'eau de brome abroge complètement leur bioactivité. Par contre les composés phosphoépoxides selon l'invention qui sont stimulants à des concentrations de l'ordre de 20 nM résistent à ces mêmes traitements chimiques, ce qui démontre la différence de structure chimique entre les composés synthétiques selon l'invention et les Tubag d'origine naturelle.

### EXEMPLE 8: Toxicité d' Epox-PP (sel de sodium):

Cinq souris de 30g ont reçu une injection intraveineuse (veine caudale) de 300 µl de tampon PBS contenant 1 mg d'Epox-PP (sel de sodium). On n'observe aucun signe de choc ou de pyrogénicité : 5 souris sont survivantes à 30 jours; aucune variation significative du poids des souris n'est notée au cours de l'étude. La toxicité est donc inférieure à 20 % pour une dose de 33,4 mg d'Epox-PP (sel de sodium) par kilogramme d'animal.

## Revendications

1. Composés comprenant au moins un groupement phosphoépoxyde de formule : 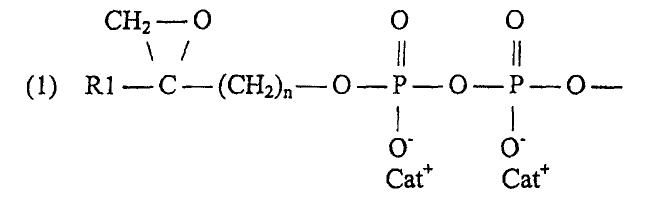 où
R1 est choisi parmi ―CH₃ et ―CH₂ ―CH₃,
Cat+ est un cation organique ou minéral,
n est un nombre entier compris entre 2 et 20,
pour leur utilisation à titre de substances thérapeutiquement actives.

2. Composés phosphoépoxydes de formule : 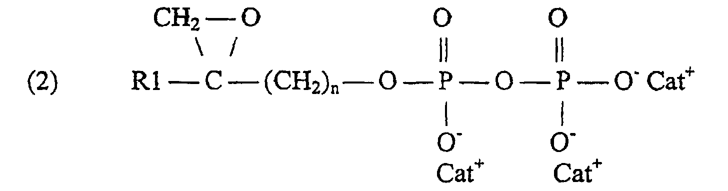 où
R1 est choisi parmi ―CH₃ et ―CH₂ ―CH₃,
Cat⁺ est un cation organique ou minéral,
n est un nombre entier compris entre 2 et 20,
pour leur utilisation à titre de substances thérapeutiquement actives.

3. Composés comprenant au moins un groupement phosphoépoxyde de formule : 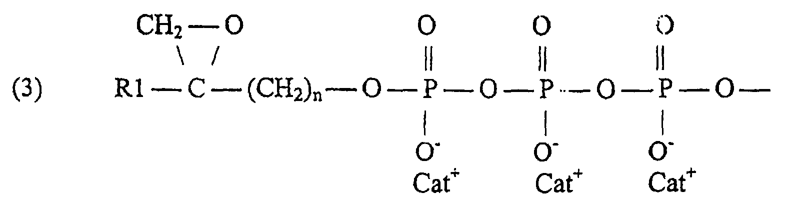 où
R1 est choisi parmi ―CH₃ et ―CH₂―CH₃
Cat⁺ est un cation organique ou minéral
n est un nombre entier compris entre 2 et 20.

4. Composés phosphoépoxydes selon la revendication 3 de formule :

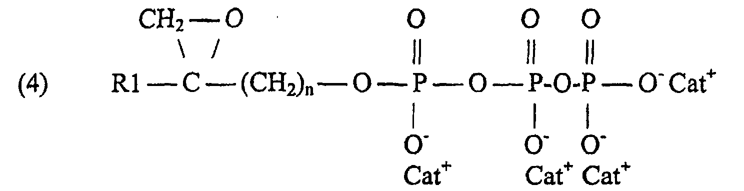

5. Composés phosphoépoxydes selon la revendication 3, de formule : 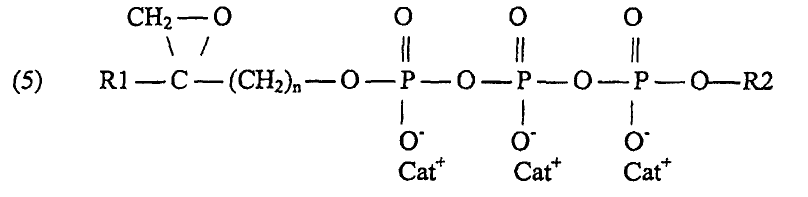 où R2 est un substituant organique ou minéral choisi dans le groupe formé :
- des substituants qui n'empêchent pas la formation de la fonction halohydrine 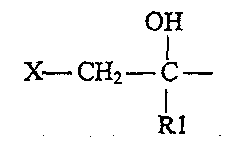 à partir de la fonction aloène 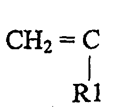 et de l'halogène X₂ en présence d'eau ;
- et des substituants pour lesquels il existe un composé R2-O-Y non réactif sur la fonction halohydrine du composé de formule : 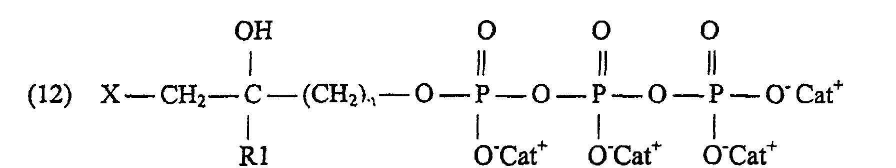 et choisi pour que R2-O-Y puisse réagir sur le phosphate terminal de ce composé (12) pour obtenir le composé (15) : 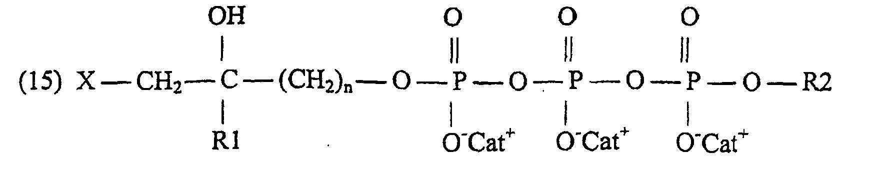
- et des substituants pour lesquels il existe un composé R2-O-PPP, où PPP symbolise le groupement triphosphate.

6. Composés selon l'une des revendications 1, 3 et 5, comprenant au moins un groupement choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des halohydrines, et des époxydes.

7. Composés selon les revendications 5 et 6, dans lesquels R2 est en outre choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des halohydrines, des phosphoépoxydes selon la formule (1), et des époxydes.

8. Composés phosphoépoxydes de formule : 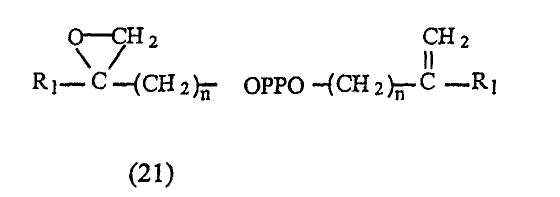 où
R1 est choisi parmi ―CH₃ et ―CH₂―CH₃,
n est un nombre entier compris entre 2 et 20.

9. Composés selon l'une des revendications 3 à 5, 7 ou 8 pour leur utilisation comme substances thérapeutiquement actives.

10. Composés selon l'une des revendications 1 à 9 pour leur utilisation comme agents activateurs des lymphocytes Tγ9δ2.

11. Composés selon l'une des revendications 1 à 10 pour leur utilisation à titre d'antigènes des lymphocytes Tγ9δ2 dans une composition thérapeutique -notamment une composition immunostimulante ou un vaccin pour les primates.

12. Procédé de fabrication d'un composé comprenant au moins un groupement phosphoépoxyde de formule : 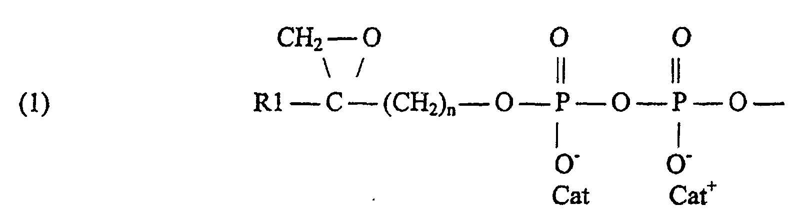 où R1 est choisi parmi ―CH₃ et ―CH₂ ―CH₃, Cat⁺ est un cation organique ou minéral, n est un nombre entier compris entre 2 et 20, **caractérisé en ce que** :
- on prépare tout d'abord un composé intermédiaire comprenant au moins un groupement phosphohalohydrine de formule : 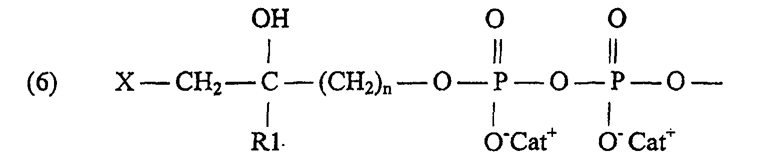
où X est un halogène choisi parmi I, Br, Cl,
- on fait réagir le composé intermédiaire avec un milieu générateur d'hydroxydes pour transformer les fonctions halohydrines du composé intermédiaire en fonctions époxydes.

13. Procédé selon la revendication 12, **caractérisé en ce que** pour préparer ledit composé intermédiaire, on fait réagir l'halogène X2 en présence d'eau avec un composé de départ comprenant au moins un groupement alcène phosphaté de formule :

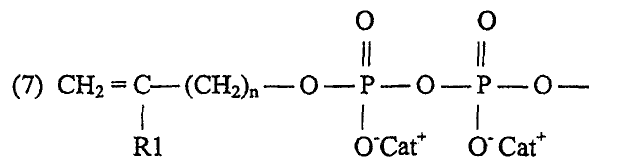

14. Procédé selon l'une des revendications 12 et 13, **caractérisé en ce qu'**on fait réagir le composé intermédiaire en milieu aqueux basique pour transformer les fonctions halohydrines du composé intermédiaire en fonctions époxydes.

15. Composition pour le diagnostic extracorporel, **caractérisée en ce qu'**elle comporte au moins un composé selon l'une des revendications 3 à 5, 7 ou 8.

16. Composition thérapeutique, **caractérisée en ce qu'**elle comporte au moins un composé selon l'une des revendications 1 à 8.

17. Composition thérapeutique, **caractérisée en ce qu'**elle comprend une quantité apte à être administrée à un primate -notamment au contact du sang périphérique ou par voie topique- d'au moins un composé selon l'une des revendications 1 à 8.

18. Composition selon l'une des revendications 15 et 17, **caractérisée en ce qu'**elle comprend en outre des lymphocytes Tγ9δ2 de primates.

19. Composition selon l'une des revendications 15 à 18, **caractérisée en ce qu'**elle comprend en outre une proportion d'interleukine adaptée pour engendrer une croissance lymphocytaire dans le milieu où elle est destinée à être administrée.

20. Procédé de fabrication d'une composition ayant la propriété d'activer les lymphocytes Tγ9δ2, dans lequel on utilise au moins un composé selon l'une des revendications 1 à 8.

21. Procédé de fabrication d'une composition thérapeutique destinée au traitement préventif ou curatif d'une pathologie produisant des cellules sensibles aux lymphocytes Tγ9δ2, dans lequel on utilise au moins un composé selon l'une des revendications 1 à 8.

22. Procédé de fabrication d'une composition thérapeutique destinée à être administrée à un primate pour le traitement préventif ou curatif d'une pathologie produisant des cellules sensibles aux lymphocytes Tγ9δ2, dans lequel on utilise au moins un composé selon l'une des revendications 1 à 8.

23. Procédé de fabrication d'une composition thérapeutique destinée à être administrée à un primate pour le traitement préventif ou curatif d'une pathologie sélectionnée dans le groupe formé des cancers, des maladies infectieuses, des parasitoses, et des pathologies à syndrome d'immunodéficience, dans lequel on utilise au moins un composé selon l'une des revendications 1 à 8.

24. Procédé selon l'une des revendications 20 à 23, dans lequel on met au moins un composé selon l'une des revendications 1 à 11 au contact d'un milieu contenant des lymphocytes Tγ9δ2 compatible avec la croissance lymphocytaire T, en une quantité adaptée pour activer les lymphocytes Tγ9δ2 dans ce milieu.

25. Procédé selon la revendication 24, dans lequel ledit milieu comprend une substance choisie parmi le sang des primates et les extraits de sang des primates.

26. Procédé d'activation extracorporelle de lymphocytes Tγ9δ2 de primates, dans lequel on met les lymphocytes Tγ9δ2 au contact d'au moins un composé tel que défini à l'une des revendications 1 à 8 dans un milieu extracorporel contenant les lymphocytes Tγ9δ2 compatible avec la croissance lymphocytaire T.

27. Procédé selon la revendication 26, dans lequel on utilise au moins un composé selon l'une des revendications 1 à 8 à une concentration dans le milieu qui procure une activation de la prolifération polyclonale des lymphocytes Tγ9δ2.

28. Procédé selon l'une des revendications 26 à 27, dans lequel on introduit dans le milieu une proportion d'interleukine adaptée pour engendrer une croissance lymphocytaire dans le milieu.

## Claims

1. Compounds comprising at least one phosphoepoxide group of the formula: 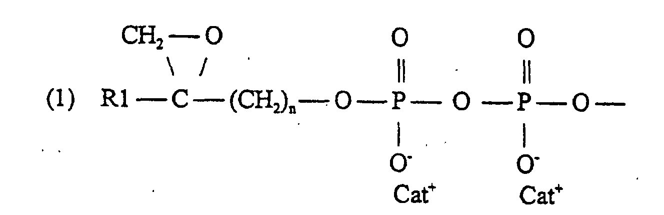 where
R1 is selected from among ―CH₃ and ―CH₂ ―CH₃,
Cat+ is an organic or inorganic cation,
n is an integer between 2 and 20,
for the use thereof as therapeutically active substances.

2. Phosphoepoxide compounds of the formula: 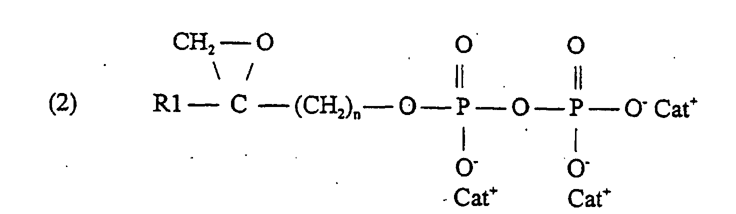 where
R1 is selected from among ―CH₃ and ―CH₂ ―CH₃,
Cat⁺ is an organic or inorganic cation,
n is an integer between 2 and 20,
for the use thereof as therapeutically active substances.

3. Compounds comprising at least one phosphoepoxide group of the formula: 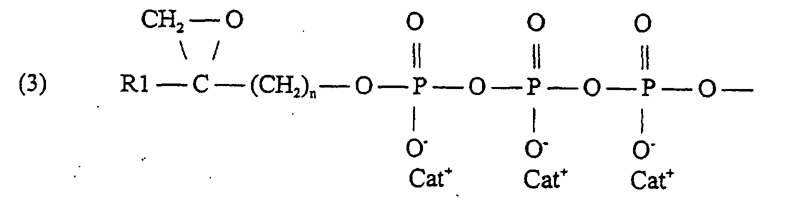 where
R1 is selected from among ―CH₃ and ―CH₂ ―CH_{3,}
Cat⁺ is an organic or inorganic cation,
n is an integer between 2 and 20.

4. Phosphoepoxide compounds as claimed in claim 3 of the formula:

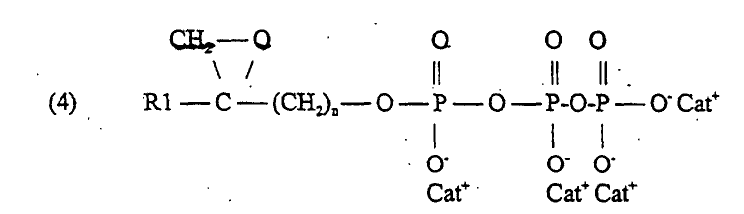

5. Phosphoepoxide compounds as claimed in claim 3 of the formula: 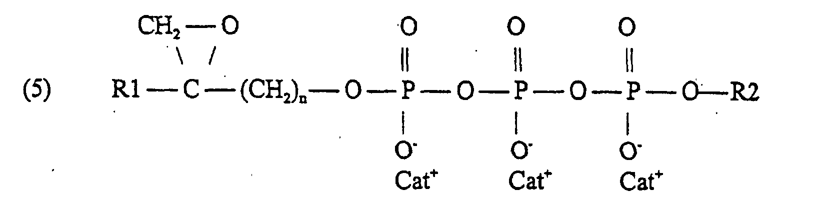 where R2 is an organic or inorganic substituent selected from among the group comprising:
- substituents which do not prevent formation of the halohydrin function 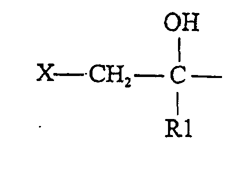 starting from the alkene function 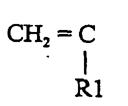 and halogen X₂ in the presence of water;
- and substituents for which there is an R2-O-Y compound which is not reactive towards the halohydrin function of the compound of the formula: 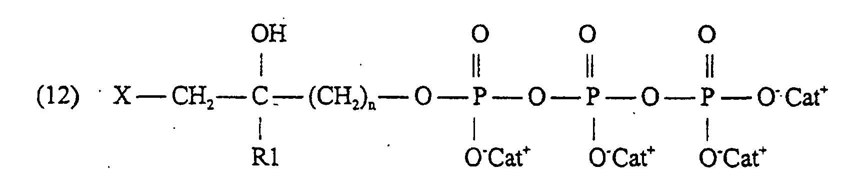 and selected such that R2-O-Y may react with the terminal phosphate of this compound (12) in order to obtain the compound (15): 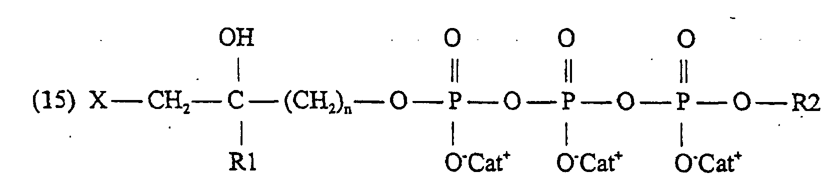
- and substituents for which there is a compound R2-O-PPP, where PPP denotes the triphosphate group.

6. Compounds as claimed in one of claims 1, 3 and 5, comprising at least one group selected from among the group comprising nucleoside derivatives, oligonucleotides, nucleic acids (RNA, DNA), amino acids, peptides, proteins, monosaccharides, oligosaccharides, polysaccharides, fatty acids, simple lipids, complex lipids, folic acid, tetrahydrofolic acid, phosphoric acids, inositol, vitamins, co-enzymes, flavonoids, aldehydes, halohydrins and epoxides.

7. Compounds as claimed in claims 5 and 6, in which R2 is moreover selected from among the group comprising nucleoside derivatives, oligonucleotides, nucleic acids (RNA, DNA), amino acids, peptides, proteins, monosaccharides, oligosaccharides, polysaccharides, fatty acids, simple lipids, complex lipids, folic acid, tetrahydrofolic acid, phosphoric acids, inositol, vitamins, co-enzymes, flavonoids, aldehydes, halohydrins, phosphoepoxides of the formula (I) and epoxides.

8. Phosphoepoxide compounds of the formula: 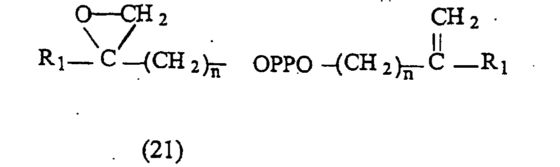 where
R1 is selected from among ―CH₃ and ―CH₂ ―CH₃,
n is an integer between 2 and 20.

9. Compounds as claimed in one of claims 3 to 5, 7 or 8 for the use thereof as therapeutically active substances.

10. Compounds as claimed in one of claims 1 to 9 for the use thereof as Tγ9δ2 lymphocyte activators.

11. Compounds as claimed in one of claims 1 to 10 for the use thereof as Tγ9δ2 lymphocyte antigens in a therapeutic composition, in particular an immunostimulant therapeutic composition or a vaccine, for primates.

12. Process for the production of a compound comprising at least one phosphoepoxide group of the formula: 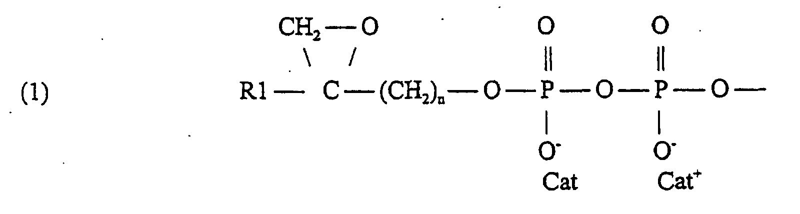 where R1 is selected from among ―CH₃ and ―CH₂―CH₃, Cat⁺ is an organic or inorganic cation, n is an integer between 2 and 20, wherein:
- an intermediate compound comprising at least one phosphohalohydrin group of the formula is first prepared: 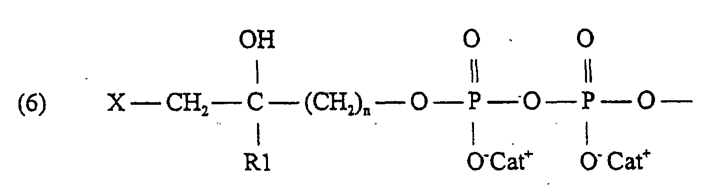
where X is a halogen selected from among I, Br, Cl,
- the intermediate compound is reacted with a hydroxide-producing medium in order to convert the halohydrin functions of the intermediate compound into epoxide functions.

13. Process as claimed in claim 12, **characterized in that**, in order to prepare said intermediate compound, the halogen X2 is reacted in the presence of water with a starting compound comprising at least one phosphorylated alkene group of the formula:

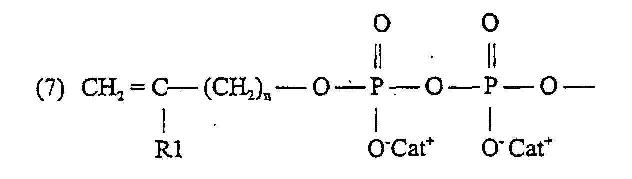

14. Process as claimed in one of claims 12 and 13, **characterized in that** the intermediate compound is reacted in a basic aqueous medium in order to convert the halohydrin functions of the intermediate compound into epoxide functions.

15. Composition for extracorporeal diagnostics, **characterized in that** it comprises at least one compound as claimed in one of claims 3 to 5, 7 or 8.

16. Therapeutic composition, **characterized in that** it comprises at least one compound as claimed in one of claims 1 to 8.

17. A therapeutic composition, **characterized in that** it comprises a quantity capable of being administered to a primate, in particular in contact with the peripheral bloodstream or topically, of at least one compound as claimed in one of claims 1 to 8.

18. The composition as claimed in one of claims 15 to 17, **characterized in that** it moreover comprises primate Tγ9δ2 lymphocytes.

19. The composition as claimed in one of claims 15 to 18, **characterized in that** it moreover comprises a proportion of interleukin suitable to bring about lymphocyte growth in the medium into which it is to be administered.

20. A process for the production of a composition having the characteristic of activating Tγ9δ2 lymphocytes, in which at least one compound as claimed in one of claims 1 to 8 is used.

21. A process for the production of a therapeutic composition intended for the preventive or curative treatment of a pathological condition which produces cells sensitive to Tγ9δ2 lymphocytes, in which process at least one compound as claimed in one of claims 1 to 8 is used.

22. A process for the production of a therapeutic composition intended to be administered to a primate for the preventive or curative treatment of a pathological condition which produces cells sensitive to Tγ 9δ2 lymphocytes, in which process at least one compound as claimed in one of claims 1 to 8 is used.

23. A process for the production of a therapeutic composition intended to be administered to a primate for the preventive or curative treatment of a pathological condition selected from among the group comprising cancers, infectious diseases, parasitic conditions, and pathological immunodeficiency syndromes, in which process at least one compound as claimed in one of claims 1 to 8 is used.

24. The process according to claim one of claims 20 to 23, in which at least one compound as claimed in one of claims 1 to 11 is brought into contact with a medium which contains Tγ9δ2 lymphocytes, and is compatible with T lymphocyte growth, in a quantity suitable for activating these Tγ9δ2 lymphocytes in this medium.

25. The process as claimed in claim 24, in which said medium comprises a substance selected from among primate blood and primate blood extracts.

26. An extracorporeal Tγ9δ2 lymphocyte activation process, in which the Tγ9δ2 lymphocytes are brought into contact with at least one compound as claimed in one of claims 1 to 8 in an extracorporeal medium which contains Tγ9δ2 lymphocytes and is compatible with T lymphocyte growth.

27. The process as claimed in claim 26, in which at least one compound as claimed in one of claims 1 to 8 is used at a concentration in the medium which brings about activation of polyclonal proliferation of Tγ9δ2 lymphocytes.

28. The process as claimed in one of claims 26 to 27, in which a proportion of interleukin suitable to bring about lymphocyte growth in the medium is introduced into the medium.

## Patentansprüche

1. Verbindungen, umfassend wenigstens eine Phosphoepoxidgruppierung der Formel: 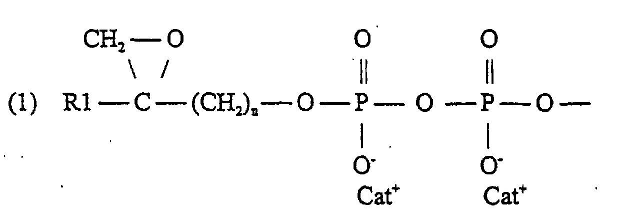 wobei
R1 aus -CH₃ und -CH₂-CH₃ ausgewählt ist,
Cat+ ein organisches oder mineralisches Kation ist
und n eine ganze Zahl zwischen 2 und 20 ist,
für die Verwendung als therapeutisch aktive Substanzen.

2. Phosphoepoxidverbindungen der Formel: 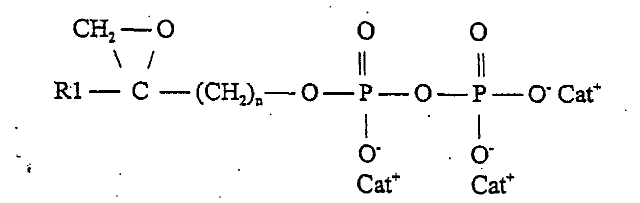 wobei
R1 aus -CH₃ und -CH₂-CH₃ ausgewählt ist,
Cat+ ein organisches oder mineralisches Kation ist,
und n eine ganze Zahl zwischen 2 und 20 ist,
für die Verwendung als therapeutisch aktive Substanzen.

3. Verbindungen, die wenigstens eine Phosphoepoxidgruppierung der folgenden Formel umfassen: 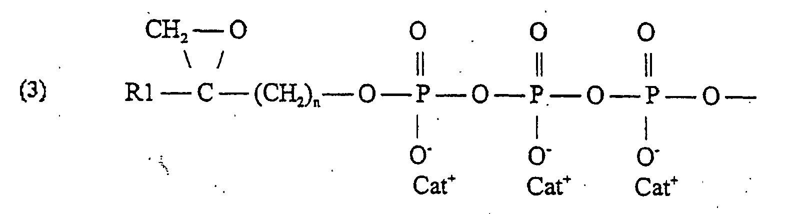 wobei
R1 aus -CH₃ und -CH₂-CH₃ ausgewählt ist,
Cat+ ein organisches oder mineralisches Kation ist,
und n eine ganze Zahl zwischen 2 und 20 ist.

4. Phosphoepoxidverbindungen nach Anspruch 3 der Formel:

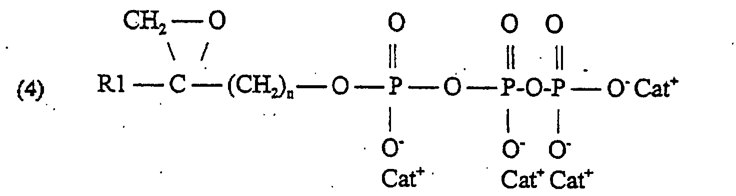

5. Phosphoepoxidverbindungen nach Anspruch 3 der Formel: 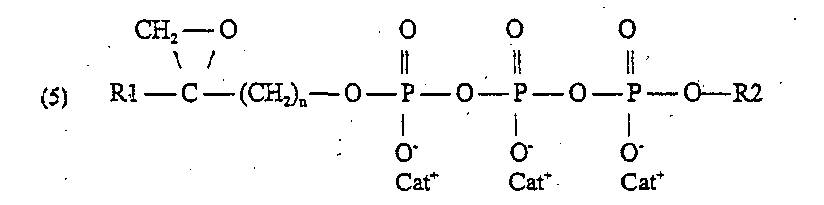 wobei R2 ein organischer oder mineralischer Substituent ist, der ausgewählt wurde aus der Gruppe bestehend aus:
- Substituenten, die die Bildung der Halohydrinfunktion 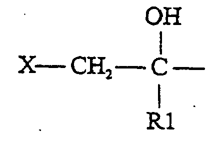 starting from the alkene function 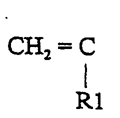 nicht behindern, und aus Wasserstoff X₂ in Anwesenheit von Wasser;
- und aus Substituenten, für die eine nichtreaktive Verbindung R2-O-Y mit der Halohydrinfunktion der Verbindung der Formel: 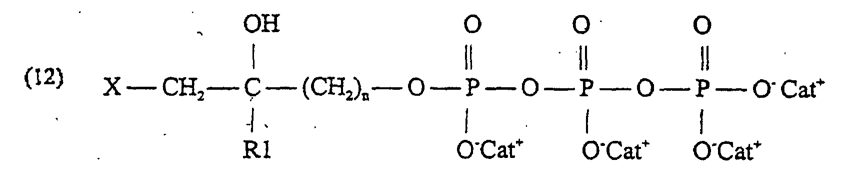 existiert, und die so gewählt wird, dass R2-O-Y auf dem Phosphatterminal dieser Verbindung (12) reagieren kann, so dass die Verbindung (15) entsteht: 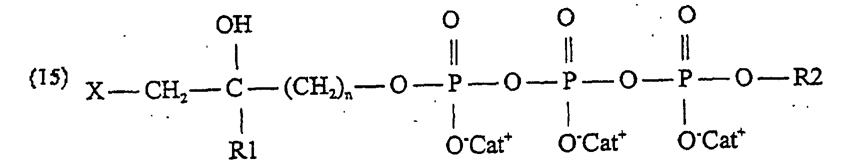
- und aus Substituenten, für die eine Verbindung R2-O-PPP existiert, wobei PPP die Triphosphatgruppierung symbolisiert.

6. Verbindungen nach einem der Ansprüche 1, 3 und 5, umfassend wenigstens eine Gruppierung ausgewählt aus der Gruppe bestehend aus Derivaten von Nukleosiden, Oligonukleotiden, Nukleinsäuren (ARN, ADN), Aminsäuren, Peptiden, Proteinen, Monosacchariden, Oligosacchariden, Polysacchariden, Fettsäuren, einfachen'Lipiden, komplexen Lipiden, Folsäure, Tetrahydrofolsäure, Phosphorsäuren, Inositol, Vitaminen, Coenzymen, Flavenoiden, Aldehyden, Halohydrinen und Epoxiden.

7. Verbindungen nach Anspruch 5 und 6, bei denen R2 darüber hinaus ausgewählt wird aus der Gruppe bestehend aus Derivaten von Nukleosiden, Oligonukleotiden, Nucleinsäuren (ARN, ADN), Aminsäuren, Peptiden, Proteinen, Monosacchariden, Oligosacchariden, Polysacchariden, Fettsäuren, einfachen Lipiden, komplexen Lipiden, Folsäure, Tetrahydrofolsäure, Phosphorsäuren, Inositol, Vitaminen, Coenzymen, Flavenoiden, Aldehyden, Halohydrinen, Phosphohalohydrinen gemäß Formel (1) und Epoxiden.

8. Phosphoepoxidverbindungen der Formel: 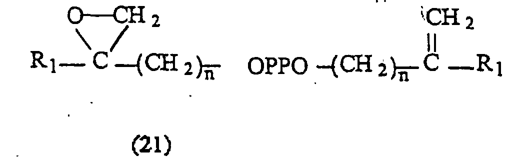 wobei
R1 aus -CH₃ und -CH₂-CH₃ ausgewählt ist,
n eine ganze Zahl zwischen 2 und 20 ist.

9. Verbindungen nach einem der Ansprüche 3 bis 5, 7 und 8 für die Verwendung als therapeutisch aktive Substanzen.

10. Verbindungen nach einem der Ansprüche 1 bis 9 für die Verwendung als Aktivator von Tγ9δ2-Lymphozyten.

11. Verbindungen nach Anspruch 1 bis 10 für die Verwendung als Antigene von Tγ9δ2-Lymphozyten in einer therapeutischen Zusammensetzung, insbesondere einer immunstimulanten Zusammensetzung oder einem Vakzin für Primate.

12. Verfahren zur Herstellung einer Verbindung, umfassend wenigstens eine Phosphoepoxidgruppierung der Formel: 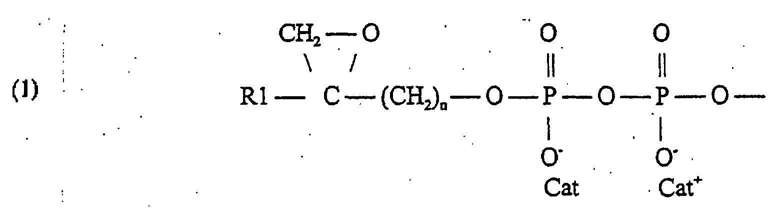 wobei R1 aus -CH₃ und -CH₂-CH₃ ausgewählt ist, Cat⁺ ein organisches oder mineralisches Kation ist, n eine ganze Zahl zwischen 2 und 20 ist, **dadurch gekennzeichnet, dass**:
- zunächst eine Zwischenverbindung hergestellt wird, die wenigstens eine Phosphohalohydrinverbindung der folgenden Formel umfasst: 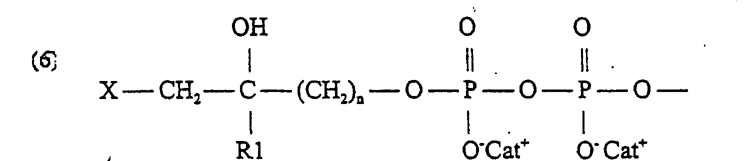
wobei X ein Halogenatom ist, ausgewählt aus I, Br, Cl,
- die Zwischenverbindung in einem Hydroxiderzeugungsmilieu reagieren gelassen wird, um die Halohydringruppen der Zwischenverbindung in Epoxidgruppen umzuwandeln.

13. Verfahren nach Anspruch 12, **dadurch gekennzeichnet, dass** zur Herstellung der genannten Zwischenverbindung Halogen X2 in Anwesenheit von Wasser mit einer Ausgangsverbindung reagieren gelassen wird, die wenigstens eine phosphatierte Alkengruppierung der folgenden Formel umfasst:

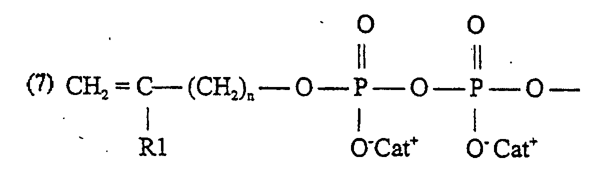

14. Verfahren nach einem der Ansprüche 12 und 13, **dadurch gekennzeichnet, dass** die Zwischenverbindung in einem basischen wässrigen Milieu reagieren gelassen wird, um die Halohydringruppen der Zwischenverbindung in Epoxidgruppen umzuwandeln.

15. Zusammensetzung für die extrakorporale Diagnostik, **dadurch gekennzeichnet, dass** sie wenigstens eine Verbindung nach einem der Ansprüche 3 bis 5, 7 und 8 aufweist.

16. Therapeutische Zusammensetzung, **dadurch gekennzeichnet, dass** sie wenigstens eine Verbindung nach einem der Ansprüche 1 bis 8 umfasst.

17. Therapeutische Zusammensetzung, **dadurch gekennzeichnet, dass** sie eine einem Primaten verabreichbare Menge, insbesondere in Kontakt mit peripherem Blut oder über einen topischen Weg, von wenigstens einer Verbindung nach einem der Ansprüche 1 bis 8 umfasst.

18. Zusammensetzung nach einem der Ansprüche 15 und 17, **dadurch gekennzeichnet, dass** sie darüber hinaus Tγ9δ2-Lymphozyten von Primaten umfasst.

19. Zusammensetzung nach einem der Ansprüche 15 bis 18, **dadurch gekennzeichnet, dass** sie ferner einen Interleukinanteil umfasst, der ein lymphozytäres Wachstum in einem Milieu erzeugen kann, in dem sie verabreicht werden soll.

20. Verfahren zur Herstellung einer Zusammensetzung mit der Eigenschaft, die Tγ9δ2-Lymphozyten zu aktivieren, in der wenigstens eine Verbindung nach einem der Ansprüche 1 bis 8 verwendet wird.

21. Verfahren zur Herstellung einer therapeutischen Zusammensetzung für eine präventive oder kurative Behandlung einer Pathologie, die für Tγ9δ2-Lymphozyten empfindliche Zellen erzeugt, bei dem wenigstens eine Verbindung nach einem der Ansprüche bis 8 verwendet wird.

22. Verfahren zur Herstellung einer therapeutischen Zusammensetzung, die einem Primaten für die präventive oder kurative Behandlung einer Pathologie verabreicht werden soll, die für Tγ9δ2-Lymphozyten empfindliche Zellen erzeugt, bei dem wenigstens eine Verbindung nach einem der Ansprüche 1 bis 8 verwendet wird.

23. Verfahren zur Herstellung einer therapeutischen Zusammensetzung, die einem Primaten für die präventive oder kurative Behandlung einer Pathologie verabreicht werden kann, ausgewählt aus der Gruppe bestehend aus Krebs, Infektionskrankheiten, Parasitosen und Pathologien mit Immunmangelsyndrom, bei dem wenigstens eine Verbindung nach einem der Ansprüche 1 bis 8 verwendet wird.

24. Verfahren nach einem der Ansprüche 20 bis 23, bei dem wenigstens eine Verbindung nach einem der Ansprüche 1 bis 11 in Kontakt mit einem Tγ9δ2-Lymphozyten enthaltenden Milieu gebracht wird, das mit dem T-Lymphozytenwachstum kompatibel ist, in einer Menge, die ausreicht, um diese Tγ9δ2-Lymphozyten in diesem Milieu zu aktivieren.

25. Verfahren nach Anspruch 24, bei dem das genannte Milieu eine Substanz umfasst, die aus Primatenblut und Extrakten von Primatenblut ausgewählt wird.

26. Verfahren zur extrakorporalen Aktivierung von Tγ9δ2-Lymphozyten von Primaten, bei dem die Tγ9δ2-Lymphozyten in einem die mit T-Lymphozytenwachstum kompatiblen Tγ9δ2-Lymphozyten enthaltenden extrakorporalen Milieu mit wenigstens einer Verbindung nach einem der Ansprüche 1 bis 8 in Kontakt gebracht werden.

27. Verfahren nach Anspruch 26, bei dem wenigstens eine Verbindung nach einem der Ansprüche 1 bis 8 in dem Milieu in einer Konzentration verwendet wird, die eine Aktivierung der polyklonalen Proliferation von Tγ9δ2-Lymphozyten bewirkt.

28. Verfahren nach einem der Ansprüche 26 bis 27, bei dem in das Milieu ein Interleukinanteil geleitet wird, der ausreicht, um ein lymphozytäres Wachstum in dem Milieu zu erzeugen.
